# EUROPEAN PATENT SPECIFICATION

(11) **EP 4 127 152 B1**
(45) Date of publication and mention of the grant of the patent: **12.08.2026**
(21) Application number: 21780901.1
(22) Date of filing: 02.04.2021
(51) Int. Cl.: C12Q 1/6806, C12N 9/10, C12N 9/22, C12N 15/09, C12Q 1/68, C40B 30/00, C40B 30/04, G01N 1/28, C12Q 1/6869

(54) **METHODS, COMPOSITIONS, AND KITS FOR IDENTIFYING REGIONS OF GENOMIC DNA BOUND TO A PROTEIN**
VERFAHREN, ZUSAMMENSETZUNGEN UND KITS ZUR IDENTIFIZIERUNG VON REGIONEN GENOMISCHER DNA, DIE AN EIN PROTEIN GEBUNDEN IST
PROCÉDÉS, COMPOSITIONS ET KITS POUR IDENTIFIER DES RÉGIONS D'ADN GÉNOMIQUE LIÉES À UNE PROTÉINE

(30) Priority: 02.04.2020 US 202063004361 P
(43) Date of publication of application: 08.02.2023
(73) Proprietor: Altius Institute For Biomedical Sciences, Seattle, WA 98121 (US); The Brigham & Women's Hospital, Inc., Boston, Massachusetts 02115 (US)
(72) Inventor: STAMATOYANNOPOULOS, John A., Seattle, Washington 98121 (US); STERGACHIS, Andrew B., Boston, Massachusetts 02115 (US)
(74) Representative: Boult Wade Tennant LLP
(86) International application number: PCT/US2021/025644
(87) International publication number: WO 2021/203047

(56) References cited:
- WO-A1-2018/227025
- WO-A1-2019/139951
- US-A1- 2010 221 716
- US-A1- 2012 115 132
- US-A1- 2017 369 855
- US-A1- 2018 265 922
- US-B1- 6 413 751
- SHIPONY ZOHAR ET AL: "Long-range single-molecule mapping of chromatin accessibility in eukaryotes", NATURE METHODS, NATURE PUBLISHING GROUP US, NEW YORK, vol. 17, no. 3, 10 February 2020 (2020-02-10), pages 319 - 327, XP037050146, ISSN: 1548-7091, [retrieved on 20200210], DOI: 10.1038/S41592-019-0730-2
- RHOADS ANTHONY ET AL: "PacBio Sequencing and Its Applications", GENOMICS PROTEOMICS AND BIOINFORMATICS, BEIJING GENOMICS INSTITUTE, BEIJING, CN, vol. 13, no. 5, 2 November 2015 (2015-11-02), pages 278 - 289, XP029335898, ISSN: 1672-0229, DOI: 10.1016/J.GPB.2015.08.002
- WENGER AARON M ET AL: "Accurate circular consensus long-read sequencing improves variant detection and assembly of a human genome", NATURE BIOTECHNOLOGY, NATURE PUBLISHING GROUP US, NEW YORK, vol. 37, no. 10, 12 August 2019 (2019-08-12), pages 1155 - 1162, XP036897227, ISSN: 1087-0156, [retrieved on 20190812], DOI: 10.1038/S41587-019-0217-9
- ANTON BRIAN P., HARHAY GREGORY P., SMITH TIMOTHY P. L., BLOM JOCHEN, ROBERTS RICHARD J.: "Comparative Methylome Analysis of the Occasional Ruminant Respiratory Pathogen Bibersteinia trehalosi", PLOS ONE, PUBLIC LIBRARY OF SCIENCE, vol. 11, no. 8, 24 August 2016 (2016-08-24), pages e0161499, XP055925794, DOI: 10.1371/journal.pone.0161499
- MURRAY IAIN A, MORGAN RICHARD D, LUYTEN YVETTE, FOMENKOV ALEXEY, CORRÊA IVAN R., DAI NAN, ALLAW MOHAMMED B, ZHANG XING, CHENG XIAO: "The non-specific adenine DNA methyltransferase M.EcoGII", NUCLEIC ACIDS RESEARCH, OXFORD UNIVERSITY PRESS, GB, vol. 46, no. 2, 25 January 2018 (2018-01-25), GB , pages 840 - 848, XP055925795, ISSN: 0305-1048, DOI: 10.1093/nar/gkx1191

## Description

### CROSS-REFERENCE TO RELATED APPLICATIONS

This application claims priority to United States Provisional Application Serial No. 63/004,361 filed April 2, 2020

### INCORPORATION BY REFERENCE OF SEQUENCE LISTING PROVIDED AS A

### TEXT FILE

A Sequence Listing is provided herewith as a text file, "ALTI-730WO Seq List_ST25.txt," created on April 2, 2020 and having a size of 11 KB. The contents of the text file are incorporated by reference herein in their entirety.

### INTRODUCTION

The primary architecture of chromatin comprises nucleosome arrays punctuated by short regulatory regions populated with transcription factors and other non-histone proteins. This architecture is foundational for genome function, yet remains undefined at the level of individual chromatin fibers - the fundamental units of gene regulation. For example, although nucleosomes present the major barrier limiting transcriptional factor access to DNA, neither the positioning nor the occupancy of nucleosomes along individual chromatin fibers *in vivo* has yet been elucidated. As such, it is currently unknown how nucleosomes are precisely ordered along the same extended chromatin template; the interplay between accessible regulatory DNA and nucleosomes on individual chromatin fibers; the extent to which a given DNA-encoded regulatory region is actuated on different chromatin fibers within a population of cells; and to what degree nearby regulatory regions are coordinately actuated on the same chromatin template. Addressing these questions requires the sequencing of individual chromatin fibers, which is not obtainable with current single cell or bulk profiling approaches.

Methods are needed for recording the primary architecture of chromatin onto its underlying DNA template at single nucleotide resolution, thereby enabling the simultaneous identification of genetic and epigenetic features along multi-kilobase segments of the genome. The present disclosure addresses these and other needs.

### SUMMARY

Methods, compositions (not claimed), kits, and systems (not claimed) are provided for identifying regions of genomic DNA bound to a protein. In certain aspects, the methods include contacting genomic DNA with an adenine methyltransferase (A-MTase), where the A-MTase causes methylation of adenine residues in regions of the genomic DNA not bound to a protein; and conducting single-molecule long-read sequencing of the contacted genomic DNA to detect locations in the genomic DNA lacking methylated adenine residues to identify regions of genomic DNA bound to a protein. In certain aspects, the bound regions are nucleosome positions. As such, encompassed by the methods are methods of determining nucleosome positions in genomic DNA. Compositions (not claimed), systems (not claimed) and kits that find use, e.g., in practicing the methods of the present disclosure are also provided.

Also provided are methods for visualization of regions of chromatin not bound to a protein and spatially available as a substrate for an adenine methyltransferase (A-MTase) in a cell by visualizing location of methylated adenines after contacting the cells with the A-MTase. The A-MTase, which is used in the claimed method and which is present in the claimed kit is Hia5. The invention is defined by the claims. Any subject-matter falling outside the scope of the claims is provided for information purposes only.

### BRIEF DESCRIPTION OF THE DRAWINGS

The invention may be best understood from the following detailed description when read in conjunction with the accompanying drawings. Included in the drawings are the following figures:
**FIGS. 1A-1F****.** Non-specific m6A-MTases selectively mark sites of chromatin accessibility.
**FIGS. 2A-2H****.** Fiber-seq exposes base-pair resolution maps of individual chromatin fiber architecture.
**FIGS. 3A-3B****.** Co-actuation of neighboring regulatory elements on the same chromatin fiber.
**FIG. 4A-4D****.** Impact of regulatory DNA actuation on nucleosome positioning.
**FIG. 5A-5E****.** Conservation of chromatin architecture between drosophila and humans.
**FIGS. 6A-6E****.** Identification of *in vivo* chromatin architectures using a cell penetrating peptide (CPP)-tagged m6A-MTase.
**FIG. 7****.** Identification of functional gene regulatory DNA alterations using Fiber-seq.
**FIG. 8****.** Punctate pattern for N6-methyladenosine (m6A) that increases with Hia5 dose as detected using three different antibodies that specifically bind to m6A
**FIG. 9****.** Hia5 dose-dependent increase in the overall nuclear m6A signal (left) as well as in the intensity of individual puncta (right).

### DETAILED DESCRIPTION

Methods, compositions, kits, and systems are provided for identifying regions of genomic DNA bound to a protein. In certain aspects, the methods include contacting genomic DNA with an adenine methyltransferase (A-MTase), where the A-MTase causes methylation of adenine residues in regions of the genomic DNA not bound to a protein; and conducting single-molecule long-read sequencing of the contacted genomic DNA to detect locations in the genomic DNA lacking methylated adenine residues to identify regions of genomic DNA bound to a protein. In certain aspects, the bound regions are nucleosome positions. As such, encompassed by the methods are methods of determining nucleosome positions in genomic DNA. Compositions, systems and kits that find use, e.g., in practicing the methods of the present disclosure are also provided. In certain aspects, at least some steps of the method are performed using a computer comprising a processor comprising programming that when executed by the processor performs the steps.

Also provided are methods for visualization of regions of chromatin not bound to a protein and spatially available as a substrate for an adenine methyltransferase (A-MTase) in a cell by visualizing location of methylated adenines after contacting the cells with the A-MTase.

Before exemplary embodiments of the present invention are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and exemplary methods and materials may now be described. Any and all publications mentioned herein are incorporated herein by reference to disclose and describe the methods and/or materials in connection with which the publications are cited. It is understood that the present disclosure supersedes any disclosure of a publication referred to herein to the extent there is a contradiction.

It must be noted that as used herein and in the appended claims, the singular forms "a", "an", and "the" include plural referents unless the context clearly dictates otherwise. Thus, for example, reference to "a cell" includes a plurality of such cells and reference to "the cell" includes reference to one or more cells, and so forth.

It is further noted that the claims may be drafted to exclude any element which may be optional. As such, this statement is intended to serve as antecedent basis for use of such exclusive terminology as "solely", "only" and the like in connection with the recitation of claim elements, or the use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed. To the extent such publications may set out definitions of a term that conflicts with the explicit or implicit definition of the present disclosure, the definition of the present disclosure controls.

### DEFINITIONS

The term "Hia5" refers to a polypeptide that is at least 80% identical (e.g., at least 85%, at least 90%, at least 95%, at least 96%, at least 97%, at least 98%, at least 99%, or a 100% identical) to the amino acid sequence of the Hia5 polypeptide (SEQ ID NO: 1) from Haemophilus influenzae (*H. influenzae*)*.*

MANQNTFKQAPLPFIGQKRMFLKQFEQILNENISDNGEGWTILDTFGGSG
LLSHTAKRLKPKARVIYNDFDGYAERLAHIDDINQLRAELYSVVGNATSKNKRMTKDC
KAECIRIIQNFKGYKDLNCLASWLLFSGQQVATLDDLFQHNFWHCIRQSDYPKADGYL
DGVEIVKESFHTLLPKFSNDPKALFVLDPPYLCTKQESYKQATYFDLIDFLRLVNITRPPY
VFFSSTKSEFIRFVNYMLEDKVDNWQAFENAKRITVNAKLNYQVAYEDNLVYKF (SEQ
ID NO:1).

The term "Hin1523" refers to a polypeptide that is at least 80% identical e.g., at least 85%, at least 90%, at least 95%, at least 96%, at least 97%, at least 98%, at least 99%, or a 100% identical) to the polypeptide (SEQ ID NO:2) encoded by *hin1523* gene from *H. influenzae.*

MSEYLEYQNAIEGKTMANKKTFKQAPLPFIGQKRMFLKHVEIVLNKHID
GEGEGWTIVDVFGGSGLLSHTAKQLKPKATVIYNDFDGYAERLNHIDDINRLRQIIFNCL
HGIIPKNGRLSKEIKEEIINKINDFKGYKDLNCLASWLLFSGQQVGSVEALFAKDFWNCV
RQSDYPTAEGYLDGIEVISESFHKLIPRYQNQDKVLLLLDPPYLCTRQESYKQATYFDLI
DFLRLINLTKPPYIFFSSTKSEFIRYLNYMQESKTDNWRAFENYKRIVVKASASKDGIYE
DNMIYKF (SEQ ID NO:2).

The term "M.Btr192IV" as used herein refers to a polypeptide that is at least 80% identical e.g., at least 85%, at least 90%, at least 95%, at least 96%, at least 97%, at least 98%, at least 99%, or a 100% identical) to the polypeptide (SEQ ID NO:3) encoded by *WQG 17550* gene from *Bibersteinia trehalosi USDA-ARS-USMARC-192*.

MKKTLTALAVASLASATQTKQQASKQASKQASKQASKECEMAKVFKQA
PLPFIGQKRMFLKHFEQVLAHIPDDGNGWTIVDVFGGSGLLSHTAKRLKPKARVIYNDY
DNYSERLQHIDDINRLRRIIADLMADTPKYKRLDNAKKLQIIEAIEAFQGYKDLHILCSW
LAFSGQQVSSFDELYKQNFWHCIRQSDYLTADGYLDGVEIVRESFHQLVPRFTGQPNTL
LVLDPPYLCTHQESYKQERYFDLVDFLRLIHLTKPPYVFFSSTKSEFVRFIDAMVEDKW
DNWQAFDDAQRIVVQTSASYNGKYEDNMVYKF (SEQ ID NO:3)

The term "EcoG1" as used herein refers to a polypeptide that is at least 80% identical e.g., at least 85%, at least 90%, at least 95%, at least 96%, at least 97%, at least 98%, at least 99%, or a 100% identical) to the polypeptide (SEQ ID NO:4) encoded by *pHK08* 22 gene from *Escherichia coli* (*E. coli*)*.*

MSRFILGDCVRVMATFPDNAVDFILTDPPYLVGFRDRSGRTIAGDVNDD
WLQPASNEMYRVLKKDALMVSFYGWNRIDRFMAAWKRAGFSVVGHLVFTKNYTSKA

AYVGYRHECAYILAKGRPALPQKPLPDVLGWKYSGNRHHPTEKPVTSLQPLIESFTHPN
AIVLDPFAGSGSTCVAALQSGRRYIGIELLEQYHRAGQQRLAAVQRAMQQGAANDNW
FEPEAA (SEQ ID NO:4)

Hia5, EcoGII, Btr192IV, and EcoGI have adenine-methyltransferase activity. These methyltransferases may be codon-optimized to increase expression in *Escherichia coli* (*E. coli*) cells.

The A-MTase such as N⁶-adenine methyltransferase (m6A-MTase) disclosed herein encompass modified Hia5, EcoGII, Btr192IV, and EcoGI, such as, variants having an amino acid sequence different from the sequences disclosed herein, mutants comprising insertions, substitutions, deletions, and fusion proteins. Fusion proteins encompass A-MTasefused to a cell-penetrating peptide, a tag, and the like. The cell-penetrating peptide may be a peptide having a net positive charge to make the fusion protein plasma membrane permeable. The cell-penetrating peptide may be HIV-1 TAT translocation domain, 8-Arginine (8R), Penetratin, variants thereof, and the like. Fusion proteins encompass A-MTase fused to a nuclear localization sequence (NLS) to target the A-MTase to the cell nucleus. An A-MTase may be fused to a NLS and a cell penetrating peptide.

The terms "antibody" and "immunoglobulin" include antibodies or immunoglobulins of any isotype, fragments of antibodies which retain specific binding to antigen, including, but not limited to, Fab, Fv, scFv, Fd, Fab', Fv, F(ab')2, chimeric antibodies, humanized antibodies, monoclonal antibodies, single-chain antibodies, including antibodies comprising only heavy chains (e.g. VHH camelid antibodies), bispecific antibodies, and fusion proteins comprising an antigen-binding portion of an antibody and a non-antibody protein. Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2. The terms "antibody" and "immunoglobulin" specifically include, without limitation, IgG1, IgG2, IgG3 and IgG4 antibodies. The antibodies may be detectably labeled, e.g., with a radioisotope, an enzyme which generates a detectable product, a fluorescent protein, and the like. The antibodies may be further conjugated to other moieties, such as members of specific binding pairs, e.g., biotin (member of biotin-avidin specific binding pair), and the like.

"Antibody fragments" comprise a portion of an intact antibody, for example, the antigen binding or variable region of the intact antibody. Examples of antibody fragments include Fab, Fab', F(ab')2, and Fv fragments; diabodies; linear antibodies (Zapata et al., Protein Eng. 8(10): 1057-1062 (1995)); single-chain antibody molecules, including antibodies comprising only heavy chains (e.g. VHH camelid antibodies); and multispecific antibodies formed from antibody fragments. Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')2 fragment that has two antigen combining sites and is still capable of cross-linking antigen.

"Single-chain Fv", "sFv" or "scFv" antibody fragments comprise the V_{H} and V_{L} domains of antibody, wherein these domains are present in a single polypeptide chain. In some embodiments, the Fv polypeptide further comprises a polypeptide linker between the V_{H} and V_{L} domains, which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994*).*

As used herein, the terms "treatment," "treating," and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment," as used herein, covers any treatment of a disease in a mammal, including in a human, and includes: (a) preventing the disease from occurring in a subject which may be predisposed to the disease but has not yet been diagnosed as having it; (b) inhibiting the disease, i.e., arresting its development; and (c) relieving the disease, i.e., causing regression of the disease.

The terms "individual," "subject," "host," and "patient," used interchangeably herein, refer to a mammal, including, but not limited to, murines (rats, mice), non-human primates, humans, canines, felines, ungulates (e.g., equines, bovines, ovines, porcines, caprines), etc.

A "biological sample" encompasses a variety of sample types obtained from an individual and can be used in a diagnostic or monitoring assay. The definition encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents, solubilization, or enrichment for certain components, such as polynucleotides. The term "biological sample" encompasses a clinical sample, and also includes cells in culture, cell supernatants, cell lysates, serum, plasma, biological fluid, and tissue samples.

### METHODS

The present disclosure provides methods for identifying regions of genomic DNA bound to a protein. The protein may be any protein that limits the access of an adenine methyltransferase (A-MTase) to an adenine base present in the genomic sequence bound by the protein. The protein may be one of more of nucleosomes, transcription factors, transcriptional repressors, and the like. Various steps and aspects of the methods will now be described in greater detail below.

Methods of the present disclosure include contacting genomic DNA with an adenine methyltransferase (A-MTase), where the A-MTase causes methylation of adenine residues in regions of the genomic DNA not bound to a protein and conducting single-molecule long-read sequencing of the contacted genomic DNA to detect locations in the genomic DNA lacking methylated adenine residues to identify regions of genomic DNA bound to a protein.

In certain aspects, the A-MTase is a N⁶-adenine methyltransferase (m6 A-MTase). In certain aspects, the m6 A-MTase is Hia5. In certain aspects, the m6 A-MTase is EcoGII. In certain aspects, m6 A-MTase is Btr192IV. In certain aspects, m6 A-MTase is EcoGI.

The contacting may involve contacting isolated genomic DNA with the A-MTase. In other aspects, the contacting may involve introducing into the cell a nucleic acid encoding the A-MTase or introducing the A-MTase into the cell.

In certain aspects, the genomic DNA is from a single cell, a plurality of cells (e.g., cultured cells), a tissue, an organ, or an organism (e.g., bacteria, yeast, or the like). In certain aspects, the genomic DNA is from a cell(s), tissue, organ, and/or the like of an animal. In some embodiments, the animal is a mammal (e.g., a mammal from the genus *Homo,* a rodent (e.g., a mouse or rat), a dog, a cat, a horse, a cow, or any other mammal of interest). In certain aspects, the genomic DNA is from a cell(s), tissue, organ, and/or the like of a human. In other aspects, the genomic DNA is from a source other than a mammal, such as bacteria, yeast, insects (e.g., drosophila), amphibians (e.g., frogs (e.g., Xenopus)), viruses, plants, or any other non-mammalian source. In certain aspects, the genomic DNA is from a cancer cell.

In some embodiments, the genomic DNA is cell-free. Such cell-free genomic DNA may present in, or obtained from, any suitable source. In certain aspects, the cell-free genomic DNA is present in or obtained from a body fluid sample selected from whole blood, blood plasma, blood serum, amniotic fluid, saliva, urine, pleural effusion, bronchial lavage, bronchial aspirates, breast milk, colostrum, tears, seminal fluid, peritoneal fluid, pleural effusion, and stool. In some embodiments, the genomic DNA is cell-free fetal DNAs. In certain aspects, the genomic DNA is circulating tumor DNAs. In some embodiments, the genomic DNA comprises infectious agent DNAs. In some embodiments, the genomic DNA comprises DNAs from a transplant. The term "cell-free genomic DNA" as used herein can refer to genomic DNA composition having no cells or substantially no cells. Genomic DNA does not necessarily imply that all of the genetic material of a cell is present, rather, genomic DNA can include a fraction of the genomic material of a cell. For example, genomic DNA encompasses isolated chromatin fragment which may be any segment of genomic DNA isolated from a cell that is in association with a nuclear protein. Exemplary chromatin fragments may be oligonucleosomes, mononucleosomes, centromeres, telomeres or genomic DNA bound by a transcription factor or chromatin remodeling factor.

In certain aspects, the cells may be peripheral blood mononuclear cells (PBMCs), leukocytes, or may be isolated from bone marrow, thymus, tissue biopsy, tumor, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. In some embodiments, the nucleic acid (e.g. genomic DNA, chromosomal DNA) to be assessed is from blood cells, e.g. blood cells from a sample of whole blood or a sub- population of cells in whole blood. Subpopulations of cells in whole blood include platelets, red blood cells (erythrocytes), platelets and white blood cells (i.e., peripheral blood leukocytes, which are made up of neutrophils, lymphocytes, eosinophils, basophils and monocytes). White blood cells can be further divided into two groups, granulocytes (which are also known as polymorphonuclear leukocytes and include neutrophils, eosinophils and basophils) and mononuclear leukocytes (which include monocytes and lymphocytes). Lymphocytes can be further divided into T cells, B cells and NK cells. Peripheral blood cells are found in the circulating pool of blood and not sequestered within the lymphatic system, spleen, liver, or bone marrow.

In certain aspects, the subject methods may involve analyzing genomic DNA obtained pre-treatment and genomic DNA obtained post-treatment. For example, after 1 day, 1 week, 10 days, 15 days, 1 month, 3 months, 6 months or more post-treatment to compare the regions of the DNA not bound by protein(s) and hence susceptible to adenine methylation. Comparison of adenine methylation pattern may be used to assess change in transcriptional profile of the genome.

In certain aspects, the subject methods may be used to generate a reference chromatin structure and regulatory regions for a type of cell. For example, for multiple types of human cells. The chromatin structure and regulatory regions in a cell from a subject having a disorder may be compared to the reference chromatin structure and regulatory regions for that cell type to determine any differences. Such differences may reveal previously unknown changes in chromatin structure and regulatory regions that may be used for diagnosis, prognosis, or treating the subject.

In some embodiments, the population of cells used for the methods may be composed of any number of cells, e.g., about 500 to about 10⁶ or more cells, about 500 to about 100,000 cells, about 500 to about 50,000 cells, about 500 to about 10,000 cells, about 50 to 1000 cells, about 1 to 500 cells, about 1 to 100 cells, about 1 to 50 cells, or a single cell. In some embodiments, the cell sample includes less than about 1000, 2000, 3000, 4000, 5000, 6000, 7000, 8000, 9000, 10,000, 15,000, 20,000, 25,000, 30,000, 40,000, 50,000, 60,000, 70,000, 80,000, 90,000, 100,000, 120,000, 140,000, 160,000, 180,000, 200,000, 250,000, 300,000, 350,000, 400,000, 450,000, 500,000, 600,000, 700,000, 800,000, 900,000, or 1,000,000 cells. In some embodiments, the cell sample includes more than about 1000, 2000, 3000, 4000, 5000, 6000, 7000, 8000, 9000, 10,000, 15,000, 20,000, 25,000, 30,000, 40,000, 50,000, 60,000, 70,000, 80,000, 90,000, 100,000, 120,000, 140,000, 160,000, 180,000, 200,000, 250,000, 300,000, 350,000, 400,000, 450,000, 500,000, 600,000, 700,000, 800,000, 900,000, or 1,000,000 cells.

In certain aspects, the genomic DNA is present in its native environment during exposure to the methyl transferase. For example, the genomic DNA may be present in a cell (e.g., an intact cell or permeabilized cell) during exposure to the methyl transferase. In some embodiments, a cell-permeable methyl transferase that crosses an intact or permeabilized cell membrane may be employed. In some embodiments, a methyltransferase may be introduced into the cell using standard techniques. In certain aspects, the genomic DNA is present in a cell lysate during exposure to the methyltransferase.

In certain aspects, the genomic DNA is part of a nucleic acid sample isolated from a cell(s), tissue, organ, and/or the like of an organism, e.g., an animal, such as a human. Approaches, reagents and kits for isolating, purifying and/or concentrating nucleic acid molecules from sources of interest are known in the art and commercially available. For example, kits for isolating DNA from a source of interest include the DNeasy^{®}, QIAamp^{®}, QIAprep^{®} and QIAquick^{®} nucleic acid isolation/purification kits by Qiagen, Inc. (Germantown, Md); the DNAzol^{®}, ChargeSwitch^{®}, Purelink^{®}, GeneCatcher^{®} nucleic acid isolation/purification kits by Life Technologies, Inc. (Carlsbad, CA); the NucleoMag^{®}, NucleoSpin^{®}, and NucleoBond^{®} nucleic acid isolation/purification kits by Clontech Laboratories, Inc. (Mountain View, CA). In certain aspects, the nucleic acid is isolated from a fixed biological sample, e.g., formalin-fixed, paraffin-embedded (FFPE) tissue. Genomic DNA from FFPE tissue may be isolated using commercially available kits - such as the AllPrep^{®} DNA/RNA FFPE kit by Qiagen, Inc. (Germantown, Md), the RecoverAll^{®} Total Nucleic Acid Isolation kit for FFPE by Life Technologies, Inc. (Carlsbad, CA), and the NucleoSpin^{®} FFPE kits by Clontech Laboratories, Inc. (Mountain View, CA).

In certain aspects, subsequent to contacting the genomic DNA with a methyltransferase and prior to the sequencing, the genomic DNA may be processed for sequencing. For example, the methods may include treating the ends of the genomic DNA to produce blunt ends. Blunting is a process by which a single-stranded overhang is either "filled in", by the addition of nucleotides on the complementary strand using the overhang as a template for polymerization, or by "chewing back" the overhang, using an exonuclease activity. DNA polymerases, such as the Klenow fragment of DNA Polymerase I and T4 DNA Polymerase may be used to fill in (5' → 3') and chew back (3' → 5'). Removal of a 5' overhang can be accomplished with a nuclease, such as Mung Bean Nuclease.

In certain aspects, genomic DNA may be sheared or enzymatically digested after treatment with the methyltransferase.

Single molecule real time sequencing systems may be applied to the detection of methylated adenine through analysis of the sequence and/or kinetic data derived from such systems. In particular, methylated adenine may alter the enzymatic activity of a nucleic acid polymerase in various ways, e.g., by increasing the time for a bound nucleobase to be incorporated and/or increasing the time between incorporation events. In certain embodiments, polymerase activity is detected using a single molecule nucleic acid sequencing technology. In certain embodiments, polymerase activity is detected using a nucleic acid sequencing technology that detects incorporation of nucleotides into a nascent strand in real time. In preferred embodiments, a single molecule nucleic acid sequencing technology is capable of real-time detection of nucleotide incorporation events. Such sequencing technologies are known in the art and include, e.g., the SMRT^{®} sequencing and nanopore sequencing technologies. For more information on nanopore sequencing, see, e.g., U.S. Pat. No. 9,175,348; U.S. Pat. No. 5,795,782; Kasianowicz, et al. (1996) Proc Natl Acad Sci USA 93(24):13770-3; Ashkenas, et al. (2005) Angew Chem Int Ed Engl 44(9):1401-4; Howorka, et al. (2001) Nat Biotechnology 19(7):636-9; and Astier, et al. (2006) J Am Chem Soc 128(5):1705-10. With regards to nucleic acid sequencing, the term "template" refers to a nucleic acid molecule subjected to template-directed synthesis of a nascent strand. A template may comprise, e.g., DNA or analogs, mimetics, derivatives, or combinations thereof, as described elsewhere herein. Further, a template may be single-stranded, double-stranded, or may comprise both single- and double-stranded regions. A modification in a double-stranded template may be in the strand complementary to the newly synthesized nascent strand, or may by in the strand identical to the newly synthesized strand, i.e., the strand that is displaced by the polymerase.

The preferred direct methylation sequencing described herein may generally be carried out using single molecule real time sequencing systems, i.e., that illuminate and observe individual reaction complexes continuously over time, such as those developed for SMRT^{®} DNA sequencing (see, e.g., P. M. Lundquist, et al., Optics Letters 2008, 33, 1026). The foregoing SMRT^{®} sequencing instrument generally detects fluorescence signals from an array of thousands of zero-mode waveguides (ZMWs) simultaneously, resulting in highly parallel operation. Each ZMW, separated from others by distances of a few micrometers, represents an isolated sequencing chamber.

Detection of single molecules or molecular complexes in real time, e.g., during the course of an analytical reaction, generally involves direct or indirect disposal of the analytical reaction such that each molecule or molecular complex to be detected is individually resolvable. In this way, each analytical reaction can be monitored individually, even where multiple such reactions are immobilized on a single substrate. Individually resolvable configurations of analytical reactions can be accomplished through a number of mechanisms, and typically involve immobilization of at least one component of a reaction at a reaction site. Various methods of providing such individually resolvable configurations are known in the art, e.g., see European Patent No. 1105529 to Balasubramanian, et al.; and Published International Patent Application No. WO 2007/041394 A reaction site on a substrate is generally a location on the substrate at which a single analytical reaction is performed and monitored, preferably in real time. A reaction site may be on a planar surface of the substrate, or may be in an aperture in the surface of the substrate, e.g., a well, nanohole, or other aperture. In preferred embodiments, such apertures are "nanoholes," which are nanometer-scale holes or wells that provide structural confinement of analytic materials of interest within a nanometer-scale diameter, e.g., ^{~}1-300 nm. In some embodiments, such apertures comprise optical confinement characteristics, such as zero-mode waveguides, which are also nanometer-scale apertures and are further described elsewhere herein. Typically, the observation volume (i.e., the volume within which detection of the reaction takes place) of such an aperture is at the attoliter (10⁻¹⁸ L) to zeptoliter (10⁻²¹ L) scale, a volume suitable for detection and analysis of single molecules and single molecular complexes.

The immobilization of a component of an analytical reaction can be engineered in various ways. For example, an enzyme (e.g., polymerase, reverse transcriptase, kinase, etc.) may be attached to the substrate at a reaction site, e.g., within an optical confinement or other nanometer-scale aperture. In other embodiments, a substrate in an analytical reaction (for example, a nucleic acid template, e.g., DNA, derivatives, and mimetics thereof, or a target molecule for a kinase) may be attached to the substrate at a reaction site. Certain embodiments of template immobilization are provided, e.g., in U.S. patent application Ser. No. 12/562,690, filed Sep. 18, 2009, now U.S. Pat. No. 8,481,264 One skilled in the art will appreciate that there are many ways of immobilizing nucleic acids and proteins into an optical confinement, whether covalently or non-covalently, via a linker moiety, or tethering them to an immobilized moiety. These methods are well known in the field of solid phase synthesis and micro-arrays (Beier et al., Nucleic Acids Res. 27:1970-1-977 (1999)). Non-limiting exemplary binding moieties for attaching either nucleic acids or polymerases to a solid support include streptavidin or avidin/biotin linkages, carbamate linkages, ester linkages, amide, thiolester, (N)-functionalized thiourea, functionalized maleimide, amino, disulfide, amide, hydrazone linkages, among others. Antibodies that specifically bind to one or more reaction components can also be employed as the binding moieties. In addition, a silyl moiety can be attached to a nucleic acid directly to a substrate such as glass using methods known in the art.

Other processing steps useful for detecting the locations of the methylated adenine in the genomic DNA using a nanopore may be employed. For example, the methods may include adding one or more nanopore sequencing adapters or subregions thereof to one or more ends of the genomic DNA. By "nanopore sequencing adapter" is meant one or more nucleic acid domains that include at least a portion of a nucleic acid sequence (or complement thereof) utilized by a nanopore sequencing platform of interest, such as a nanopore sequencing platform provided by Oxford Nanopore Technologies, e.g., a MinION^{™}, GridIONx5^{™}, PromethION^{™}, or SmidgION^{™} nanopore-based sequencing system. Nanopore sequencing adapters of interest may be added via chemical or enzymatic ligation, or any other available approaches for joining one or more nucleic acid molecules to one or more ends of the double-stranded nucleic acid molecule. Suitable reagents (e.g., ligases) and kits for performing ligation reactions are known and available, e.g., the Instant Sticky-end Ligase Master Mix available from New England Biolabs (Ipswich, MA). Ligases that may be employed include, e.g., T4 DNA ligase (e.g., at low or high concentration), T4 DNA ligase, T7 DNA Ligase, *E. coli* DNA Ligase, Electro Ligase^{®}, or the like. Conditions suitable for performing the ligation reaction will vary depending upon the type of ligase used.

On certain aspects, single-molecule, circular consensus sequencing (CCS) may be used to generate accurate long read sequences. In certain aspects, CCS may involve rendering the DNA topologically circular, and sequencing the DNA multiple times in order to create a consensus sequence. In certain aspects, the circular DNA may be sequenced up to 20 times, e.g., 5-20 times, 5-15 times, 10-20, or 10-15 times.

Prior to the sequencing, the genomic DNA may be processed to generate long fragments, e.g., up to 100 kb long, up to 50 kb long, up to 40 kb long, up to 30 kb long, up to 30 kb long, up to 10 kb long, up to 5 kb long, up to 1 kb long. For example, the fragments that are sequenced may range in length from 1 kb-100 kb, 1 kb-50 kb, 1 kb-40 kb, 1 kb-30 kb, or 1 kb-20 kb.

In certain aspects, the locations of methylated adenines are detected in a contiguous stretch of a strand of the double-stranded nucleic acid molecule of 500 bases or greater, 1 kilobase (kb) or greater, 2 kb or greater, 3 kb or greater, 4 kb or greater, 5 kb or greater, 6 kb or greater, 7 kb or greater, 8 kb or greater, 9 kb or greater, 10 kb or greater, 15 kb or greater, 20 kb or greater, 25 kb or greater, 30 kb or greater, 35 kb or greater, 40 kb or greater, 45 kb or greater, 50 kb or greater, 55 kb or greater, 60 kb or greater, 65 kb or greater, 70 kb or greater, 75 kb or greater, 80 kb or greater, 85 kb or greater, 90 kb or greater, 95 kb or greater, or 100 kb or greater.

Computational approaches (e.g., in the form of software) may be employed to detect the locations of the methylated adenines in single and/or double-stranded nucleic acid molecule, determine protein bound regions in the nucleic acid molecule based on the detected locations of methylated adenines, sequence the nucleic acid molecule, e.g., using single molecule real time sequencing, and optionally, CCS, and any combinations thereof.

As summarized above, encompassed by the methods for determining bound regions in genomic nucleic acid molecules are methods for determining nucleosome positions in genomic DNA. Such methods exploit the protected/inaccessible nature of nucleosome-associated genomic DNA from the methylase (e.g., a N6-adenine DNA methyltransferase) employed, such that methylation does not occur in nucleosome-associated genomic DNA. The methods include detecting location of methylated adenine in genomic DNA that mark the locations of linker genomic DNA in the genomic DNA. The nucleosome positions in the genomic DNA are determined based on the absence of methylated adenines. Such method may also reveal presence or absence of certain transcription factors bound to genomic DNA. Also Shipony, Z. et al. (Nature Methods vol. 17, no. 3, pages 319-327 (2020)) describe a method for the long-range single-molecule mapping of chromatin accessibility in eukaryotes. The method is based is based on combining the preferential methylation of open chromatin regions by DNA methyltransferases with low sequence specificity, in this case EcoGII, an N-6-methyladenosine (m(6)A) methyltransferase, and the ability of nanopore sequencing to directly read DNA modifications.

The methods disclosed herein may be conducted on one or a plurality of normal cells to generate a chromatin accessibility map for the region of genomic DNA sequenced, where the map indicates regions of chromatin not bound to protein(s) and hence accessible to the A-MTase and regions of the chromatin bound to protein(s) and hence inaccessible to the A-MTase.

The methods disclosed herein may be conducted on one or a plurality of test cells to generate a chromatin accessibility map for genomic DNA of the test cell(s). The test cells may be from a subject, such as a mammal, e.g., a human patient. The subject, in some cases, may have or may be suspected of having a disease. The disease may be cancer.

The methods disclosed herein may further include comparing the chromatin accessibility map for the test cell to that of the normal cell, wherein the test cell and the normal cell are of the same cell type and comparing the genomic DNA sequences of the test and normal cells, wherein presence of a difference in chromatin accessibility maps indicates a change in chromatin architecture in the test cell, wherein presence of a difference in genomic DNA sequence in absence of a difference in chromatin accessibility maps indicates that the sequence difference is not associated with a change in chromatin structure, and wherein presence of a difference in genomic DNA sequence and of a difference in chromatin accessibility maps indicates the sequence difference is associated with a change in chromatin structure.

The methods further comprise generating a database comprising information regarding chromatin accessibility map, the underlying genomic DNA sequence, and correlation, if any, to a condition or disease. In certain aspects, the normal cell and test cell may be epithelial cells, white blood cells, glial cells, osteoblasts, or chondrocytes. In certain aspects, the normal cell and the test cell comprises plurality of cells. In certain aspects, the plurality of cells comprises at least 10 cells, at least 30 cells, at least 100 cells, at least 300 cells, or at least 10,000 cells.

In certain aspects, the chromatin accessibility map encompasses at least 10% of a chromatin, e.g., at least 30%, at least 50%, or at least 80% of a chromatin. In certain aspects, the chromatin accessibility map encompasses at least 10% of the genome of the cell. In certain aspects, the chromatin accessibility map encompasses at least 20%, at least 30%, at least 50%, or at least 80% of the genome of the cell. In certain aspects, the protein(s) bound to the genomic DNA includes nucleosomes, transcriptional regulator such as, transcriptional repressors and transcriptional activators, or both.

Also provided herein are methods for visualization of regions of chromatin not bound to a protein and spatially available as a substrate for an adenine methyltransferase (A-MTase) in a cell. For example, the regions of the chromatin spatially available as a substrate for the A-MTase may be regions of genomic DNA not bound to histones and/or transcription regulators (e.g., activator or repressor). The method may include contacting the cell with the A-MTase; and detecting presence of methylated adenines in the cell.

Visualization of the methylated adenines in the cells using the methods disclosed herein may be used to generate a visual map of the regulatory genome at the single cell level by selective fluorescent labeling of methylated adenines (m6A) in the chromatin of intact cells. The method may be used to visualize cells in a high-throughput manner. For example, at least 10, 100, 1000, 10,000, 100,000, 1 million, 3 million, 10 million, 30 million, 100 million or more cells may be analyzed by the disclosed methods.

The m6A imaging method may further include detection of DNA and proteins targets inside the cells. For example, the method may include multiplex detection of mA with other DNA and protein targets inside the cells.

The method may include generating a quantitative, image-based representation of the regulatory state of a cell. The method may further include analyzing the image in different cells and/or same type of cells at different time points.

The methods may include generating a quantitative image of pattern of methylated adenines present in cells from a tissue sample comprising or suspected of comprising diseased cells and comparing the pattern to a pattern representative of a normal cell.

The methods may include generating a quantitative image of pattern of methylated adenines present in cells having received a stimuli such as therapeutic drugs, and comparing the pattern to the pattern for the cells prior to such stimuli.

The cell may be any cell of interest, such as, a mammalian cell, a human cell, a T cell, B cell, diseased cell, e.g., cancer cell. The cell may be a cell as described herein.

In certain aspects, a plurality of same type of cell may be contacted with A-MTase. For example, the plurality of cells may be epithelial cells, white blood cells, glial cells, osteoblasts, or chondrocytes. The cells may be from a single individual and in some embodiments from a single tissue, such as, pancreas, blood, skin, intestine, etc.

The visualization method may include performing click-chemistry to label the methylated adenines prior to the detecting. The click-chemistry may add a fluorescent label to the methylated adenines. The visualization method may include adding a labeled methyl group as a substrate of the A-MTase. The labeled methyl group may be fluorescently labeled. Alternatively, the A-MTase may be labeled, e.g., a fluorophore conjugated version of the methyltransferase enzyme may be used.

In some embodiments, detecting presence of methylated adenine in the cell comprises contacting the cell with an antibody that specifically binds to methylated adenine. The antibody may be detectably labeled. The detectable label may be a fluorophore. The method may further include staining genomic DNA in the cell.

In some embodiments, the method may further include contacting the cell with fluorescent labeling moieties to target other specific genomic regions or cellular proteins of interest.

In some embodiments, the method may further include contacting the cell with an antibody that specifically binds to RNA polymerase II (Pol II), e.g., Pol II Ser5Phos or Pol II Ser2Phos.

In some embodiments, the method may further include measuring average nuclear intensity of and/or nuclear spot intensity of a signal specific for methylated adenine. In some embodiments, the signal specific for methylated adenine may be a fluorescent signal from a fluorescently labeled antibody bound directly or indirectly to the mA.

In some embodiments, the A-MTase is a m6 A-MTase, such as, Hia5, EcoGII, Btr192IV, or EcoGI. In some embodiments, detecting presence of methylated adenine in the cell comprises detecting m6 A.

In certain aspects, visualization of mA in genomic DNA may be used to generate a reference mA pattern for a type of cell. For example, for multiple types of human cells. The mA pattern in a cell from a subject having a disorder may be compared to the reference mA pattern for that cell type to determine any differences. Such differences may reveal previously unknown changes in chromatin structure and regulatory regions that may be used for diagnosis, prognosis, or treating the subject. The reference mA pattern may include additional information such as presence or absence of certain transcription regulators, RNA polymerases, etc.

In some embodiments, the population of cells used for the methods may be composed of any number of cells, e.g., about 500 to about 106 or more cells, about 500 to about 100,000 cells, about 500 to about 50,000 cells, about 500 to about 10,000 cells, about 50 to 1000 cells, about 1 to 500 cells, about 1 to 100 cells, about 1 to 50 cells, or a single cell. In some embodiments, the cell sample includes less than about 1000, 2000, 3000, 4000, 5000, 6000, 7000, 8000, 9000, 10,000, 15,000, 20,000, 25,000, 30,000, 40,000, 50,000, 60,000, 70,000, 80,000, 90,000, 100,000, 120,000, 140,000, 160,000, 180,000, 200,000, 250,000, 300,000, 350,000, 400,000, 450,000, 500,000, 600,000, 700,000, 800,000, 900,000, or 1,000,000 cells. In some embodiments, the cell sample includes more than about 1000, 2000, 3000, 4000, 5000, 6000, 7000, 8000, 9000, 10,000, 15,000, 20,000, 25,000, 30,000, 40,000, 50,000, 60,000, 70,000, 80,000, 90,000, 100,000, 120,000, 140,000, 160,000, 180,000, 200,000, 250,000, 300,000, 350,000, 400,000, 450,000, 500,000, 600,000, 700,000, 800,000, 900,000, or 1,000,000 cells.

Detecting presence of methylated adenine (mA, e.g., m⁶A) in the cell may involve visualization of an antibody bound to the mA. The visualization may be epifluorescence imaging when using a fluorescence label bound directly or indirectly to the antibody. A super-resolution microscopy method may be utilized for visualization of an antibody bound to the mA. The super-resolution microscopy method may be a deterministic super-resolution microscopy method, which utilizes a fluorophore's nonlinear response to excitation to enhance resolution. Exemplary deterministic super- resolution methods may include stimulated emission depletion (STED), ground state depletion (GSD), reversible saturable optical linear fluorescence transitions (RESOLFT), and/or saturated structured illumination microscopy (SSIM). A super-resolution microscopy method may also include a stochastic super-resolution microscopy method, which utilizes a complex temporal behavior of a fluorophore, to enhance resolution. Exemplary stochastic super-resolution method may include super-resolution optical fluctuation imaging (SOFI), all single-molecular localization method (SMLM) such as spectral precision determination microscopy (SPDM), SPDMphymod, photo-activated localization microscopy (PALM), fluorescence photo-activated localization microscopy (FPALM), stochastic optical reconstruction microscopy (STORM), and dSTORM.

The detecting may include generating a map of spatial location of the methylated adenines in the genome of the cell. The detecting may include generating a map of spatial and temporal location of the methylated adenines in the genome of the cell.

The method may include contacting a plurality of cells of the same type with the A-MTase and generating a map of spatial location of the methylated adenines in the genome of the cells.

The method may include contacting a plurality of cells of the same type at at least two different time points with the A-MTase and generating a map of spatial and temporal location of the methylated adenines in the genome of the cells. The two different time points may include a first time point and a second time point, wherein the first and second time points are separated by a time point at which a therapy is administered to the cells. The cells may be obtained from a subject and wherein the subject is administered the therapy.

The cells visualized by the disclosed methods may be live cells or fixed and permeabilized cells.

### SYSTEMS

The present disclosure also provided systems which find use, e.g., in practicing the subject methods, including carrying out one or more of any of the steps described above in the Methods section of the present disclosure.

Encompassed by the systems that find use in determining bound regions in genomic DNA are systems that find use in determining nucleosome positions in genomic DNA. The instructions for such systems cause the system to sequence genomic DNA that has been treated with an adenine methylase, and record the locations of the methylated adenines in the genomic DNA. The instructions for such systems may further cause the system to assess transcriptional accessibility of certain regions of the genome based on the determined positions of methylated adenines in the genomic DNA. The instructions for such systems may further cause the system to assess differential nucleosome occupancy or phasing near the promoters of genes.

The systems may be adapted (e.g., include instructions) to sequence a contiguous stretch of the genomic DNA of 500 bases or greater, 1 kilobase (kb) or greater, 2 kb or greater, 3 kb or greater, 4 kb or greater, 5 kb or greater, 6 kb or greater, 7 kb or greater, 8 kb or greater, 9 kb or greater, 10 kb or greater, 15 kb or greater, 20 kb or greater, 25 kb or greater, 30 kb or greater, 35 kb or greater, 40 kb or greater, 45 kb or greater, 50 kb or greater, 55 kb or greater, 60 kb or greater, 65 kb or greater, 70 kb or greater, 75 kb or greater, 80 kb or greater, 85 kb or greater, 90 kb or greater, 95 kb or greater, or 100 kb or greater, and record the locations of such methylated adenines.

In some embodiments, the system include a sequencing device such as a commercially available sequencer, e.g., PacBio sequencer.

The present disclosure includes computer-readable medium, including non-transitory computer-readable medium, which stores instructions for methods, or portions thereof, described herein, and which may be part of the systems of the present disclosure. Aspects of the present disclosure include computer-readable medium storing instructions that, when executed, cause the system to perform one or more steps of a method as described herein.

In some aspects instructions in accordance with the methods and systems described herein can be coded onto a computer-readable medium in the form of "programming", where the term "computer-readable medium" as used herein refers to any storage or transmission medium that participates in providing instructions and/or data to a computer for execution and/or processing. Examples of storage media include a floppy disk, hard disk, optical disk, magnetooptical disk, CD-ROM, CD-R, magnetic tape, non-volatile memory card, ROM, DVD-ROM, Blue-ray disk, solid state disk, and network attached storage (NAS), whether or not such devices are internal or external to the computer. A file containing information can be "stored" on computer-readable medium, where "storing" means recording information such that it is accessible and retrievable at a later date by a computer.

Any steps of the methods or those carried out by the systems of the present disclosure can be executed using programming that can be written in one or more of any number of computer programming languages. Such languages include, for example, Java (Sun Microsystems, Inc., Santa Clara, CA), Visual Basic (Microsoft Corp., Redmond, WA), and C++ (AT&T Corp., Bedminster, NJ), as well as any many others.

### KITS

Also provided by the present disclosure are kits. The kits include one or more reagents useful in practicing the methods of the present disclosure. In certain aspects, the kits include any reagents, devices, instructions (e.g., present on one or more non-transitory computer-readable medium), etc., useful for practicing the methods of the present disclosure, including any reagents, devices, instructions, etc. described above in the Methods and Systems sections of the present disclosure.

In some embodiments, provided is a kit that includes an adenine methylase (e.g., a N6-adenine DNA methyltransferase) that methylates adenine in genomic DNA and thereby mark the locations of unbound regions of the genomic DNA (e.g. unbound by proteins), and instructions for using the methylase in a method for determining bound regions in genomic DNA by detecting the locations of methylated adenines in the genomic DNA by using single molecule sequencing.

Components of the kits may be present in separate containers, or multiple components may be present in a single container. A suitable container includes a single tube (e.g., vial), one or more wells of a plate (e.g., a 96-well plate, a 384-well plate, etc.), or the like.

The kits may include instructions, e.g., for using the adenine methyltransferase in a method for determining bound regions in genomic DNA by detecting the locations of methylated adenines by using a sequencer. In some embodiments, the kits include instructions for using the adenine methyltransferase in a method for determining nucleosome positions in genomic DNA based on the locations of methylated adenines.

The instructions may be recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g., portable flash drive, DVD, CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, the means for obtaining the instructions is recorded on a suitable substrate.

### UTILITY

The methods, kits, and systems disclosed herein find use in generation of a database comprising information on chromatin architecture, genomic DNA sequences, and correlation of the chromatin architecture to presence or absence of a particular condition or disease. The information may be used for disease diagnosis and/or prognosis.

In certain aspects, the database may include information regarding changes to the chromatin architecture in response to a treatment as compared to prior to the treatment. In certain aspects, the information may be used to monitor efficacy of treatment and to make any adjustments to the treatment, if needed. The treatment may be an immunotherapy, e.g., an antibody therapy or treatment with a small molecule. The treatment may be for cancer.

### EXAMPLES

As can be appreciated from the disclosure provided above, the present disclosure has a wide variety of applications. Accordingly, the following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Those of skill in the art will readily recognize a variety of noncritical parameters that could be changed or modified to yield essentially similar results. Thus, the following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, dimensions, etc.) but some experimental errors and deviations should be accounted for.

### Materials and Methods

**Isolation and cloning of m6A-MTases.** pHia5ET and pHinET were kindly provided by Monika Radlinska (M. Drozdz, et al., Nucleic Acids Res. 40, 2119-2130 (2012)). Codon optimized versions of Btr192IV (GenBank:CP003745) and EcoGI (GenBank:AFST01000004) were synthesized as gBlocks by IDT and were cloned into the pET vector above using NdeI and XhoI restriction sites to generate pBtr192IVET and pEcoGIET vectors respectively. Cloning was performed in 5-alpha F'Iq competent Escherichia coli cells (NEB C2992H).

**MTase protein production.** pHia5ET, pHinET, pBtr192IVET and pEcoGIET vectors were transformed into T7 Express lysY competent Escherichia coli cells (NEB C3010I) for recombinant protein expression. Overnight cultures were added to two 1 L cultures of LB medium supplemented with 100 µg ml-1 ampicillin and grown with shaking at 37°C to an OD600 of 0.8-1.0. Isopropyl β-D-1-thiogalactopyranoside (IPTG) was added at a final concentration of 1mM and grown for an additional 4 h at 20°C with shaking. Cells were pelleted at 5000 x g for 10 min at 4°C (all subsequent steps at 4°C) and resuspended in 35 ml lysis buffer (50 mM HEPES, pH 7.5; 300 mM NaCl; 10% glycerol; 0.5% Triton X-100; 10 mM β-mercaptoethanol) supplemented with 2X Complete, EDTA-free Protease Inhibitor Cocktail (Roche 11873580001). Cells were lysed by probe sonication (Qsonica Q125) for 10 min on ice at 50% amplitude, 30 seconds on/off (20 min elapsed) then centrifuged for 1 hr at 40,000 x g. Ni-NTA Agarose (Qiagen 30210) was prepared by washing 5 mLs of slurry with 30 mLs equilibration buffer (50 mM HEPES, pH 7.5; 300 mM NaCl; 20 mM imidazole) and centrifuged at 500 x g for 3 min, repeating once. The clarified cell lysate and agarose were combined and rotated at 4°C for one hr before pouring the column. The column was washed with 20 mLs of buffer 1 (50 mM HEPES, pH 7.5; 300 mM NaCl; 50 mM imidazole) and 15 mLs of buffer 2 (50 mM HEPES, pH 7.5; 300 mM NaCl; 70 mM imidazole) before adding 15 mLs of elution buffer (50 mM HEPES, pH 7.5; 300 mM NaCl; 250 mM imidazole). The eluate was added to a 10K Amicon Ultra-15 tube and centrifuged at 3220 x g for 15 minute increments to exchange EB buffer with 15 mLs of protein resuspension buffer (50 mM Tris pH 7.5; 50 mM KCl; 1mM DTT; 10 mM EDTA; 2X Complete, EDTA-free Protease Inhibitor Cocktail). The volume was decreased below 500 µl with several 15 min spins and transferred to a 1.5 mL Eppendorf LoBind tube. The protein was supplemented with filter sterilized BSA solution to 200 µg/mL and 30% glycerol and stored at -20°C.

**In vitro MTase activity assessment.** Substrate DNA was prepared by PCR of K562 genomic DNA with primers for a 759 base pair region in the Hydroxymethylbilane Synthase (HMBS) gene promoter that contained 4 GATC sequences. The PCR fragment was purified with Monarch PCR & DNA Cleanup Kit (NEB T1030S) according to manufacturer's instructions. A series of eleven 60 µl MTase reactions were prepared with 1 µg of substrate DNA and alternating two-fold and five-fold enzyme dilutions (10, 5, 1, 0.5, 0.1, 0.05, 0.01, 0.005, 0.001, 0.0005, and 0.0001 µl MTase) in Buffer A (15 mM Tris, pH 8.0; 15 mM NaCl; 60 mM KCl; 1mM EDTA, pH 8.0; 0.5 mM EGTA, pH 8.0; 0.5 mM Spermidine) supplemented with 0.8 mM S-adenosyl-methionine (NEB B9003S). A negative control was prepared without MTase. The reactions were mixed by gentle flicking of the PCR strip tubes and a quick spin down before a 1 hr incubation at 37°C. Each reaction was stopped with Monarch PCR & DNA Cleanup Kit and the purified DNA eluted in 20 µl EB buffer. Twelve restriction enzyme digests were prepared by combining 15 µl of each purified DNA sample with 1 µl DpnI (NEB R0176S) and 4 µl of 10X CutSmart Buffer (NEB) in a 40 µl reaction. The reactions were carefully mixed by flicking and incubated at 37°C for 1.5 hours. A microliter of each reaction was combined with 2 µl of 6X Purple Gel Loading Dye (NEB B7024S) and 11 µl H2O and ran on a 1.2% agarose gel containing 1X GelGreen Nucleic Acid Stain (Biotium 41005) at 130 V for approximately 1.5 hours. The gel was imaged on a GE Typhoon FLA 9500 laser scanner. MTase activity was determined by the highest MTase dilution that methylates 1 µg of DNA substrate leading to no fully intact DNA molecules after DpnI digestion.

**MTase-seq.** Drosophila S2 cells were grown in 1X Schneider's Drosophila Medium (Gibco 21720-024) supplemented with 10% HI FBS (Gibco 16140-063) and 1% Pen Strep (Gibco 15140-122) at room temperature in 75 cm2 flasks to approximately 90% confluency and >95% viability. Cells were rinsed with PBS and then resuspended in PBS and counted on a Countess Automated Cell Counter. Three million Drosophila S2 cells per sample were pelleted at 250 x g for 5 minutes. The cell pellet was resuspended in a volume of Buffer A corresponding to 60 µl per sample and aliquoted into PCR strip tubes. To the tubes, 60 µl of cold 2X Lysis buffer (0.1 % IGEPAL CA-630 in Buffer A) was added and mixed by gentle flicking then kept on ice for 10 minutes. Samples were pelleted at 4°C for 5 min at 350 x g and the supernatant was removed. K562 and Hela nuclei were isolated as previously described (R. E. Thurman, et al., Nature. 489, 75-82 (2012)). The nuclei pellets were gently resuspended individually with wide bore pipette tips in 57.5 µl Buffer A and moved to a 37°C thermocycler. 1 µl of MTase diluted in Buffer A and 1.5 µl SAM (.8 mM final) were added, then carefully mixed by pipetting the volume up and down 10 times with wide bore tips and a multichannel pipette. The reactions were incubated for 20 minutes then stopped with 3 µl of 20% SDS (1% final) and transferred to new 1.5 mL microfuge tubes. The sample volumes were increased by adding 130 µl Buffer A and an additional 7 µl of 20% SDS. All samples were mixed with 2 µl RNase A (Invitrogen AM2271) and incubated for 1 hour at 37°C then mixed with 2 µl Proteinase K (NEB P8107S) and incubated at 55°C for an additional hour. The DNA was purified by adding 200 µl (1:1) of phenol:chloroform:isoamyl alcohol 25:24:1 (saturated with 10mM Tris, pH 8.0, 1mM EDTA) then mixed by vigorous tube inversions and incubated for 10 min at RT. Extractions were centrifuged for 10 min at 17,900 x g and the upper aqueous phase was transferred to new microfuge tubes. Residual phenol was removed with a second extraction by adding 200 µl chloroform:isoamyl alcohol 24:1 to all samples and repeating the extraction procedure. The aqueous phase was transferred to new microfuge tubes and the DNA precipitated by adding 0.1 volumes of 3M sodium acetate, 1 µl GlycoBlue Coprecipitant (Invitrogen AM9515), and 2.5 volumes ice cold 100% ethanol. All samples were inverted several times followed by a quick spin down and storage at -20°C overnight. The DNA was pelleted by centrifuging at 20,000 g for 10 min at 4°C and washed by repeating the centrifugation with 1 mL of ice cold 70% ethanol. The tubes were inverted over a tube rack and air dried for 15 minutes before resuspension in 54 µl of 10 mM Tris, pH 7.5.

All samples were transferred to MicroTUBE-50 AFA Fiber Screw-Cap sonication tubes (Covaris 520166) and sonicated individually on a Covaris M220 Focused-Ultrasonicator (peak power: 75.0; duty factor: 15.0%; cycles/burst: 200; duration: 720 seconds; water bath temperature: 20°C) to achieve a fragment length of approximately 100 base pairs. Samples were then run on a 1.2% agarose gel containing 1X GelGreen Nucleic Acid Stain and excised bands were purified with a QIAquick Gel Extraction Kit (28704). 2 duplicate samples were then pooled to get enough input for library construction and quantified by Qubit dsDNA HS Assay Kit (Invitrogen, Q32851). All libraries were prepared using the NEBNext Ultra II DNA Library Prep Kit for Illumina (NEB E7645S) and NEBNext Multiplex Oligos for Illumina Index Primers Sets 1 and 2 (NEB E7335S & E7500S). The DNA was end repaired by combing 1 µg of gel extracted DNA in 50 µl of Buffer EB (10 mM Tris-Cl, pH 8.5) with 7 µl of End Prep Reaction Buffer and 3 µl of End Prep Enzyme Mix in PCR strip tubes. Samples were mixed then placed on a thermocycler and ran with the end repair program (30 minutes at 20°C, 30 minutes at 65°C, then hold at 4°C). To end repaired samples, 2.5 µl of the Adaptor for Illumina was added followed by 30 µl of the Ligation Master Mix, then 1 µl of the Ligation Enhancer. Samples were then incubated for 15 min at 20 °C, after which 3 µl of USER enzyme was added and the samples were incubated at 37 °C for 15 minutes. 116 µl (1.2X) of Agencourt AMpure XP beads (Beckman Coulter A63880) were added to each library, followed by a 10 min incubation at room temperature and magnetic separation for 10 min. The supernatant was discarded and beads were washed twice on the magnet with 200 µl of fresh 80% ethanol followed by a 4 min air dry. Samples were removed from the magnet and 50 µl of 10 mM Tris, pH 7.5 was added to each sample followed by a 10 min incubation at room temperature. After magnetic separation for 10 min the supernatant was transferred to new LoBind tubes.

20 µl of 100 µM blocking oligo (AGATCGGAAGAGCGTC (SEQ ID NO:5)) was added to each library and the sample was incubated at 95°C for 10 minutes to denature the DNA followed by transfer to an ice/water slush mix for 10 minutes for rapid cooling. To each sample, 325 µl 0.1X TE buffer, 100 µl 5X IP Buffer (50 mM Tris, pH 7.5; 750 mM NaCl; 0.5% IGEPAL CA-630), and 5 µl (5 µg) anti-N6-methyladenosine antibody (Millipore Sigma ABE572) were added. Samples were incubated for 12 hrs on a rotator at 4°C. 25 µl of Protein A Dynabeads (Invitrogen 10001D) per sample were prepared by removing supernatant after magnetic separation and washing 4 times in 1X IP buffer (10 mM Tris, pH 7.5; 150 mM NaCl; 0.1% IGEPAL CA-630) followed by resuspending in 25 µl 1X IP buffer. 25 µl of the prepared Protein A beads was then added to each sample followed by a 4 hours incubation at 4°C rotating. Samples were then washed with 750 µl 1X IP buffer six times, with each wash consisting of a 5 min incubation with the wash buffer at 4°C rotating. DNA was eluted by adding 48 µl of Proteinase K Digestion Buffer (20 mM HEPES, pH 7.5; 1 mM EDTA; 0.5% SDS) and 2 µl proteinase K to the beads and incubating at 50°C for 1 hr with 1200 rpm of shaking. The sample was then separated using a magnet and the supernatant was transferred to a new tube. 90 µl (1.8X) of AMpure XP beads were added to each sample following the same procedure above but with elution in 17 µl 10 mM Tris, pH 7.5 followed by quantification with the Qubit ssDNA Assay Kit (Invitrogen, Q10212). Libraries were amplified by adding 5 µl of the Universal PCR primer, 5 µl of Index primer (both provided in NEBNext Multiplex Oligos for Illumina Index Primers Sets 1 and 2), and 25 µl NEBNext Ultra II Q5 Master Mix to each sample followed by PCR amplification (Program: 98°C of 30 seconds; Five to seven cycles of 98°C for 10 seconds then 65°C for 75 seconds; Final 65°C incubation for 5 min). 60 µl (1.2X) of AMpure XP beads were added to each sample following the same procedure above but with elution in 33 µl of 10 mM Tris, pH 7.5. Libraries were quantified using a Qubit dsDNA HS Assay Kit quantification and size distribution was check on an Agilent Bioanalyzer High Sensitivity DNA chip. Libraries were sequenced using an Illumina HiSeq 4000 to a read depth of 10 to 30 million reads using paired end 76bp read lengths.

**DNaseI-seq**. Drosophila S2 cells were grown as above and nuclei were isolated as above. Nuclei were then incubated with for 3 min at 37°C with limiting concentrations of DNaseI (Sigma) in buffer A supplemented with Ca2+. The digestion was stopped with stop buffer (50 mM Tris - HCl, 100 mM NaCl, 0.1% SDS, 100 mM EDTA, 1 mM spermidine, 0.5 spermine, pH 8.0) and the samples were treated with proteinase K and RNase A. The small 'double - hit' fragments (<750 bp) were recovered using AMpure XP beads and samples were prepared using an Illumina library kit as previously described (S. John, et al., Current Protocols in Molecular Biology (John Wiley & Sons, Inc., Hoboken, NJ, USA, 2013; vol. Chapter 27, pp. 21.27.1-21.27.20). Previously published K562 and Hela DNaseI-seq datasets were used for analysis (R. E. Thurman, et al., 2012 *(supra)).*

**MTase-seq and DNaseI-seq analysis.** Reads were mapped to the dm6 genome as previously described (J. Vierstra, et al., Science (80). 346, 1007-1012 (2014)). Signal tracks were generated using BEDOPS (S. Neph, et al., Bioinformatics. 28, 1919-20 (2012)) and signal was normalized to 1 million reads. Regions of chromatin accessibility (hotspots) were identified using the hotspot algorithm (S. John, et al., Nat. Genet. 43, 264-268 (2011)) at an FDR 5% cutoff. For each hotspot quantified using S2 cell DNaseI data, we quantified the total number of normalized reads contained within that hotspot to identify the signal intensity of that element. This same process was repeated using each of the MTase-seq libraries. When comparing libraries to each other, we used the S2 DNaseI-seq hotspot calls as the list of genomic regulatory elements, and quantified the signal intensity within these regions across different libraries as above. Promoter-proximal elements were defined as those within +/-500bp of a transcriptional start site as annotated within the NCBI RefSeq-Curated gene list.

**m6A dot-blot.** DNA from Drosophila S2 cell nuclei treated with different concentrations of MTase was isolated as above and samples were quantified by Nanodrop. Dilutions of these DNA samples were made in 20X SSC buffer in a 96-well plate followed by denaturation at 95°C for 10 min. Nitrocellulose membrane was wetted in 20X SSC buffer then secured in a HYBRI-DOT Manifold (Life technologies). After securing the membrane in the manifold the vacuum was applied and 150 µl of 20X SSC buffer was added to all wells followed by the denatured DNA samples. After removing any air bubbles, the vacuum was stopped and the membrane was placed face up on dry Whatman filter paper and crosslinked with 125 mJoule in a GS Gene Linker UV Chamber (Bio-Rad) using the C-L setting. The membrane was then washed with 20 mLs of 1X TBS-T (10 mM Tris, pH 7.5; 0.25 mM EDTA; 150 mM NaCl; 0.1% TWEEN-20) and blocked in 15 mLs of 1X TBS-T + 5% non-fat dry milk for 1 hour at RT. Rabbit polyclonal anti-N6-methyladenosine antibody (Millipore Sigma ABE572) was diluted 1:1000 in 10 mLs 1X TBS-T + 5% non-fat dry milk and incubated with the blot overnight at 4°C on a slow shaker. The blot was washed 3 times in 20 mLs of 1X TBS-T for 15 minutes. The anti-rabbit IgG, HRP-linked secondary antibody (Cell Signaling Technology 7074) was diluted 1: 1000 in 10 mLs 1X TBS-T + 5% non-fat dry milk and incubated with the blot for 1 hour at room temperature. Three washes were repeated and the blot was developed with Pierce ECL Plus Western Blotting Substrate (Thermo Scientific 32132) and imaged with film.

Fiber-seq. Nuclei from 2.5 million Drosophila S2 cells or 300,000 Human K562 cells were isolated as above and the MTase reaction was performed as above with the exception of using twenty units (1 µl) of Hia5. An untreated Drosophila S2 replicate was also generated for comparison. DNA was purified as above and transferred to MicroTUBE-50 AFA Fiber Screw-Cap sonication tubes (Covaris 520166) and sonicated on a Covaris M220 Focused-Ultrasonicator (peak power: 75.0; duty factor: 5.0%; cycles/burst: 200; duration: 8 seconds; water bath temperature: 20°C) to achieve a fragment length of approximately 1.5 Kb. The sheared sample was used to generate a Pacific Biosystems sequencing library using the Sequel^{®} Sequencing Kit 3.0 (Pacific Biosciences, Menlo Park, CA, USA). Small library fragments were removed using the BluePippin (Sage Science, Beverly, MA, USA) and the each library was loaded onto a single SMRTcell.

Fiber-seq m6A methylation calling. Reads were mapped to the dm6 genome using PBAlign and subreads from each ZMW of a library were extracted using bamsieve to generate a per-ZMW bam file. These per-ZMW bam files were then processed by ipdSummary using -identify m6A. For drosophila S2 analysis, p-value cutoff of 0.001 was used for methylation calling and reads with 14 or fewer subreads were discarded. To get longer reads in the human sample, for the K562 analysis, a p-value cutoff of 0.02 (i.e. phred score >16) was used for methylation calling and reads with fewer than 10 subreads were discarded. Promoter locations within each read were annotated using the NCBI RefSeq-Curated gene list and the expression of each gene was recorded using previously published RNA-seq data (43). DNaseI hypersensitive elements (DHSs) were demarcated using the hotspot calls described above and repeats were defined using RepeatMasker. Unless otherwise indicated, mitochondrial reads and reads with greater than 25% overlap with repetitive elements were removed from analysis, with the exception of K562 data where reads with greater than 50% overlap with repetitive elements were removed.

Methylase-accessible DNA sequences (MADs) identification. Per-read m6A methylation events identified above were grouped together to identify MTase-sensitive regions. Specifically, for each m6A, all m6A events within 50bp of it were joined together to call larger methylase-accessible DNA sequences (MADs). MTase-Protected Sites (MPSs) were defined as regions along each read that were not contained within an MADs. Identification of MADs overlapping DHSs was performed by identifying all MADs overlapping a DHS and then capturing the widest MADs. This widest MADs was used to identify if a DHS was closed or open/accessible on an individual read. When comparing overlapping reads, the above process was repeated for each of the overlapping reads, and the median difference in MADs size was calculated on each read for each DHS was captured.

Quantification of nucleosome phasing/positioning. The position of each nucleosome was defined as the center of each MPS between 65 and 200 bases in width. All reads overlapping this position were identified, and the position of each nucleosome on these overlapping reads was similarly defined. To calculate the phasing of a nucleosome, the distance between the nucleosome position on one read and the nearest nucleosome position on each overlapping read was identified. If more than one overlapping read was present, the median distance was used. Positioning of nucleosomes relative to TSSs was performed by first identifying TSSs that overlap DHSs and then identifying the largest MADs that overlaps these DHSs. The position of MPSs relative to this MADs was then identified relative to the reading frame of the gene. Reads overlapping 2 or more TSSs were removed from this analysis. The relative phasing of nucleosomes at each of these positions was calculated by dividing the number of nucleosomes with an offset of 0-39bp by the number of nucleosomes with an offset of 40-79bp and taking the log2 transformation of this number.

### EXAMPLE 1: NON-SPECIFIC M6A-MTASES SELECTIVELY MARK SITES OF CHROMATIN ACCESSIBILITY

The primary architecture of chromatin comprises nucleosome arrays punctuated by short regulatory regions populated with transcription factors and other non-histone proteins. This architecture is foundational for genome function, yet remains undefined at the level of individual chromatin fibers - the fundamental units of gene regulation. For example, although nucleosomes present the major barrier limiting transcriptional factor access to DNA, neither the positioning nor the occupancy of nucleosomes along individual chromatin fibers in vivo has yet been elucidated. As such, it is currently unknown how nucleosomes are precisely ordered along the same extended chromatin template; the interplay between accessible regulatory DNA and nucleosomes on individual chromatin fibers; the extent to which a given DNA-encoded regulatory region is actuated on different chromatin fibers within a population of cells; and to what degree nearby regulatory regions are coordinately actuated on the same chromatin template. Addressing these questions requires the sequencing of individual chromatin fibers, which is not obtainable with current single cell or bulk profiling approaches.

A method for recording the primary architecture of chromatin onto its underlying DNA template at single nucleotide resolution was developed, thereby enabling the simultaneous identification of genetic and epigenetic features along multi-kilobase segments of the genome. Current approaches to mapping chromatin and regulatory architectures sample large populations of chromatin fibers, and rely on dissolution of chromatin using nucleases such as DNase I (D. S. Gross, W. T. Garrard, Annu. Rev. Biochem. 57, 159-97 (1988), R. E. Thurman, et al., 2012 *(supra)),* micrococcal nuclease (M. Noll, R. D. Kornberg, J. Mol. Biol. 109, 393-404 (1977), D. E. Schones, et al., Cell. 132, 887-898 (2008)), restriction enzymes (E. Lieberman-Aiden, et al., Science (80). 326, 289-293 (2009)), transposases (J. D. Buenrostro, et al., Nat. Methods. 10, 1213-1218 (2013)), or mechanical shearing (T. Kouzarides, Cell. 128, 693-705 (2007)). CpG and GpC methyltransferases are capable of marking accessible cytosines in a dinucleotide context without digesting DNA (T. K. Kelly, et al., Genome Res. 22, 2497-2506 (2012), A. R. Krebs, et al., Mol. Cell. 67, 411-422.e4 (2017)). However, the average resolution is low due to sporadic occurrence of CpG and GpC dinucleotides in animal genomes, their linear clustering by mutation and selection, and by the confounding influence of endogenous cytosine methylation machineries (A. P. Bird, Nature. 321, 209-13 (1986)).

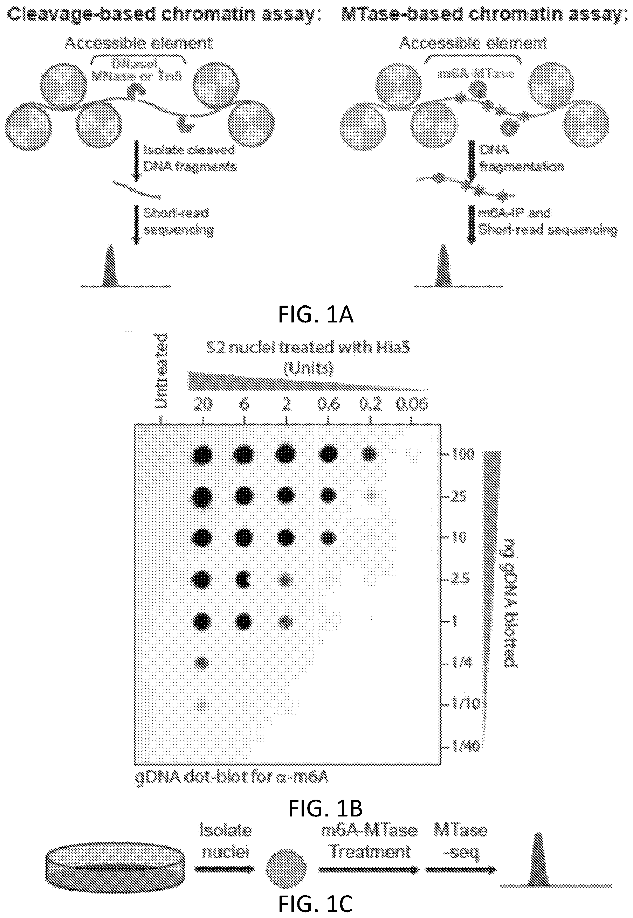

Unlike cytosine, adenine bases in DNA are almost completely devoid of endogenous methylation in eukaryotes (Q. Xie, et al., Cell. 175, 1228-1243.e20 (2018)), and occur at an average frequency approaching 1 in every 2 DNA base pairs in animal genomes without the clustering and extended deserts characteristic of cytosine-guanine dinucleotides. Therefore, non-specific (i.e., non-sequence context dependent) N⁶-adenine DNA methyltransferases (m6A-MTase) with high efficiency, high stability, and a molecular weight similar to non-specific nucleases such as DNaseI (~30kD) that are able to access protein-DNA interfaces at nucleotide resolution were sought (Fig. 1A). Five distinct non-specific DNA m6A-MTases (M. Drozdz, et al., Nucleic Acids Res. 40, 2119-2130 (2012), B. P. Anton, et al., PLoS One. 11, e0161499 (2016), I. A. Murray, et al., Nucleic Acids Res. 46, 840-848 (2018), G. Fang, et al., Nat. Biotechnol. 30, 1232-1239 (2012)) were isolated and that treatment of non-chromatinized and chromatinized DNA templates (Fig. 1B) with increasing amounts of each enzyme resulted in monotonic increases in adenine methylation, compatible with single-hit kinetics, was demonstrated.

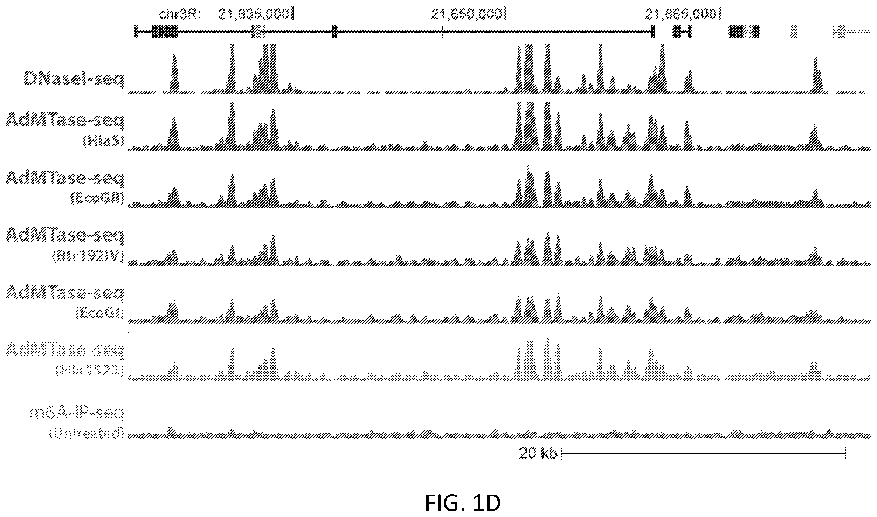

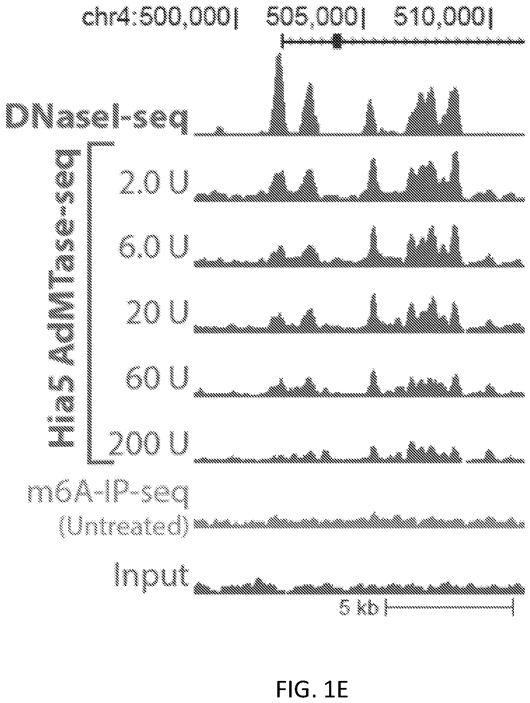

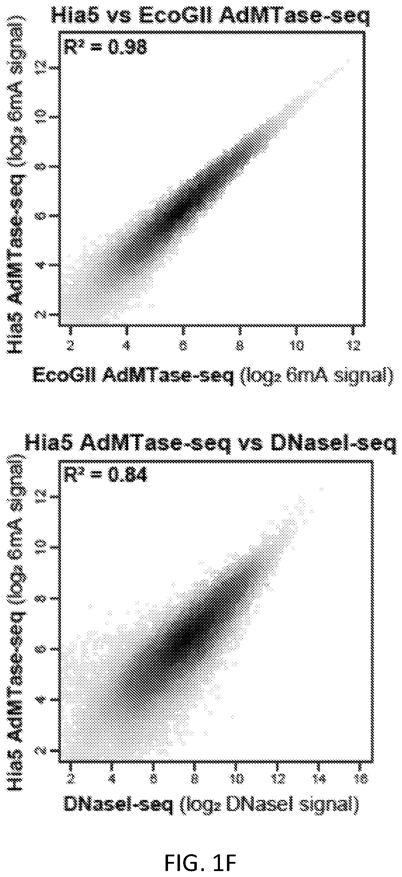

To establish selectivity of m6A-MTases for accessible DNA templates within nuclear chromatin, the distribution of DNase I cleavage (the established standard for marking accessible DNA templates (D. S. Gross, W. T. Garrard, 1988 *(supra),* R. E. Thurman, et al., *2012 (supra))* following treatment of *D. melanogaster* S2 cell nuclei with DNase I with the distribution of m6A-DNA after exposure of S2 nuclei to increasing concentrations of five adenine methyltransferases was compared (Fig. 1C). Immunoprecipitation and massively parallel sequencing of short (median 110 bp) DNA fragments harboring m6A from extracted genomic DNA (MTase-seq) showed the genomic distribution of m6A-DNA to mirror the density of DNase I cleavages quantified by DNase-seq (Fig.1D). m6A-MTases showed high selectivity for accessible DNA and quantitative representation of DNase I hypersensitive sites (DHSs) (Fig. 1D). Further, m6A-MTases demonstrated decreasing selectivity towards DHSs with increasing amounts of enzyme (Fig. 1E), analogous with the enzymatic action of DNase I (or micrococcal nuclease (MNase)) on chromatin substrates due to increasing digestion of the far more numerous but shorter internucleosomal linker regions (H. Weintraub, M. Groudine, Science (80). 193, 848-856 (1976), K. S. Bloom, J. N. Anderson, Cell. 15, 141-150 (1978), J. Mieczkowski, et al., Nat. Commun. 7, 11485 (2016)). Quantification of DNA accessibility by MTase-seq was highly reproducible at both promoter-proximal and distal regulatory elements (Fig. 1F), with the enzyme Hia5 demonstrating the highest efficiency. These results show that non-specific m6A-MTases provide quantitative probes of DNA accessibility within chromatin, and indicate that m6A is distributed at near-nucleotide resolution, effectively stenciling chromatin structure onto DNA.

FIGS. 1A-1F. Non-specific m6A-MTases selectively mark sites of chromatin accessibility. Fig. 1A. Schematic of cleavage- and m6A-MTase-based methods for marking sites of chromatin accessibility. Fig. 1B. Dot-blot quantification of m6A-modified DNA from D. *melanogaster* S2 nuclei after treatment with different amounts of the m6A-MTase Hia5. Fig. 1C. Experimental schematic for MTase-seq. Figs. 1D-1E. Genomic locus comparing the relationship between DNaseI-seq signal and MTase-seq signal after treatment of S2 cell nuclei with 5 separate m6A-MTases (Fig. 1D) or increasing amount of the m6A-MTase Hia5 (Fig. 1E). m6A-IP-seq on untreated nuclei and input control are displayed for comparison. Fig. 1F. Comparison of MTase-seq signal for S2 cell DHSs from cells treated with Hia5 versus MTase-seq signal from cells treated with EcoGII (top) or DNaseI-seq signal (bottom).

### EXAMPLE 2: FIBER-SEQ EXPOSES BASE-PAIR RESOLUTION MAPS OF INDIVIDUAL CHROMATIN FIBER ARCHITECTURE

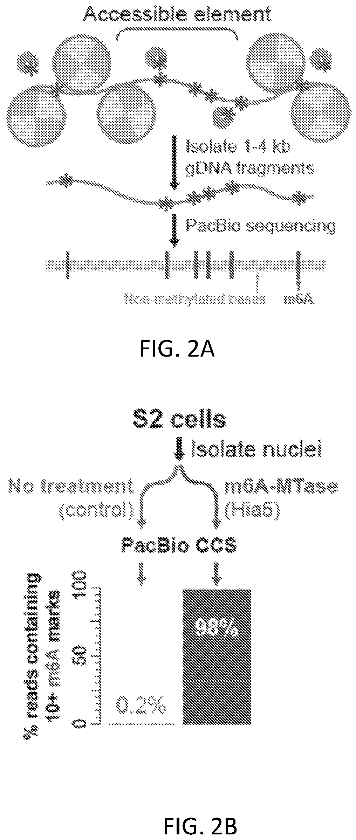

Sequencing of the linear pattern of m6A along multi-kilobase chromatin stencils at nucleotide resolution was attempted with the aim to reconstruct the primary architecture of chromatin fibers; this process was termed 'Fiber-seq' (Fig. 2A). To implement Fiber-seq, the ability of single-molecule DNA sequencers to discriminate methylated from unmethylated adenine residues based on the DNA polymerase kinetics at that base during sequencing was capitalized upon (G. Fang, et al., Nat. Biotechnol. 30, 1232-1239 (2012)). Highly accurate nucleotide resolution was achieved by re-sequencing of each chromatin fiber over 15 times (termed circular consensus sequencing, CCS (K. J. Travers, et al., Nucleic Acids Res. 38 (2010), doi: 10.1093/nar/gkq543)), enabling base calling of modified nucleotides with an accuracy comparable to unmodified nucleotides.

To create chromatin stencils, m6A-MTase treatment of S2 cell nuclei (using similar conditions as MTase-seq) was performed, followed by PCR-free library construction on high molecular weight DNA extracted from either treated or untreated nuclei. The resulting libraries were subjected to CCS on a Pacific Biosystems single molecule DNA sequencer, providing very high base calling accuracy. Whereas untreated nuclei demonstrated minimal m6A signal, over 98% of single molecule reads from m6A-MTase treated cells showed some degree of adenine methylation (Fig. 2B), and 51% of m6As had another m6A mark within four nucleotides. These results, together with those above, indicate the ability of Fiber-seq to translate chromatin stencils into a linear readout of DNA accessibility.

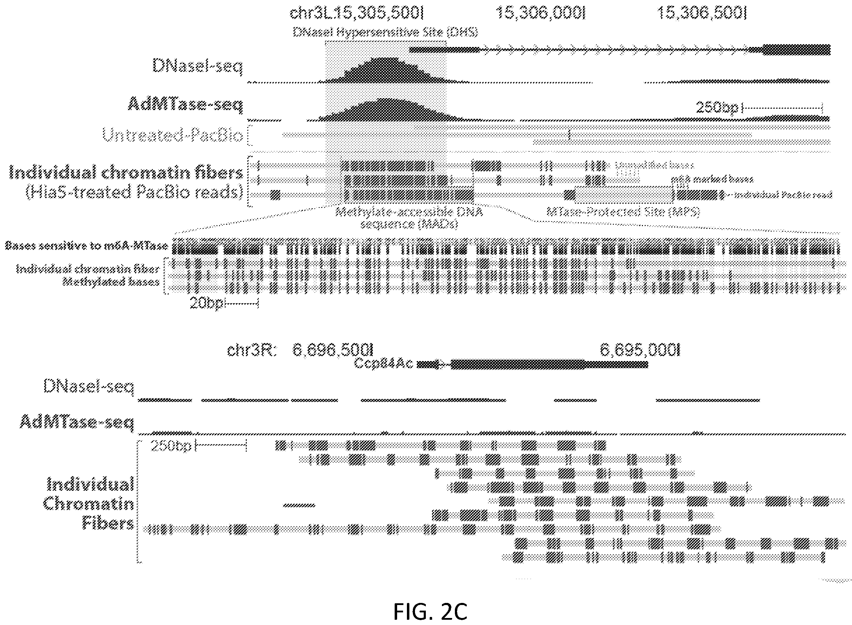

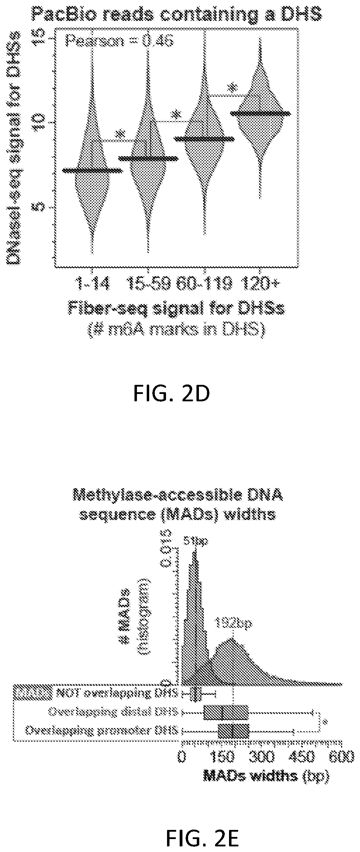

To reconstruct the primary architecture of chromatin, the distribution of m6A nucleotides along the genome was analyzed. Marked clustering of m6A into short contiguous regions spanning tens to hundreds of base pairs, separated by extended stretches of unmodified nucleotides was observed (Fig. 2C). Two categories of methylase-accessible DNA sequences (MADs) were identified: (1) sequence elements with an average length of 174bp that coincided with DNaseI hypersensitive sites (Fig. 2C,D); and (2) far more numerous shorter sequence elements with an average length of 51bp and regularized spacing, paralleling the expected size and distribution of internucleosomal linker regions (R. V. Chereji, et al., Nucleic Acids Res. 44, 1036-1051 (2016)) (Fig. 2C,D). In the first category, the aggregate number of m6A-marked bases from all fibers overlapping DHSs was proportional with aggregate density of DNase I cleavages obtained from bulk nuclei (Fig. 2E).

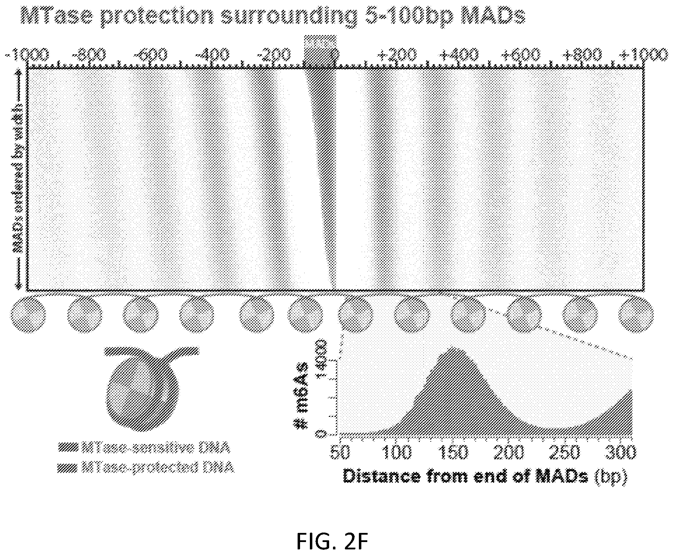

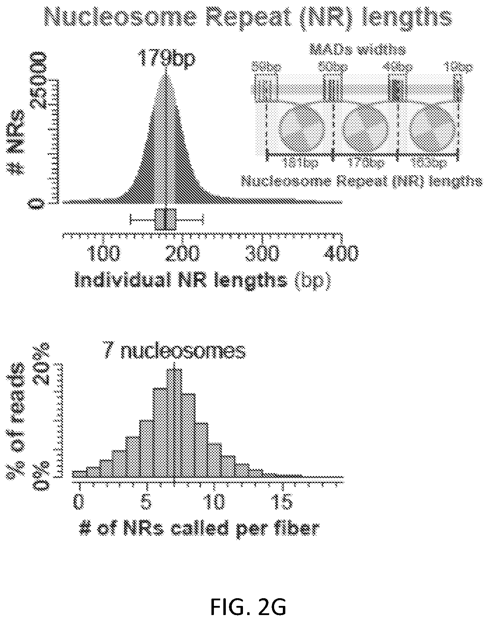

Nucleosome-occupied DNA could be readily defined by the striking lack of m6A between strongly marked linker regions, indicating that m6A-MTases are generally unable to access nucleosome-wrapped DNA (Figs. 2C,F), likely due to the fact that these enzymes modify adenine via base-flipping (J. R. Horton, et al., J. Mol. Biol. 358, 559-570 (2006)). Fiber-seq data precisely recorded nucleosome positions for up to several kilobases, with the average high-quality fiber sequence yielding >7 well-demarcated nucleosomes (Fig. 2G) enabling us to evaluate key features of nucleosome occupancy.

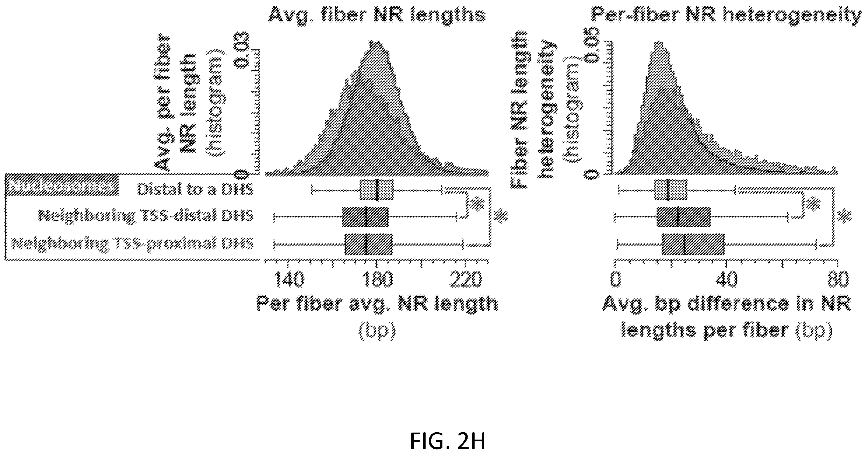

Fiber-seq data was next used to probe several fundamental questions surrounding chromatin structure and the interplay between nucleosome occupancy and regulatory DNA accessibility. An average nucleosome repeat (NR) length of 179bp was observed, consistent with prior reports (R. V. Chereji, et al., 2016 (*supra*))*.* However, the NR lengths on individual fibers varied surprisingly, with 75% ranging between 157 and 202bp in length (Fig. 2G). It has been generally assumed that the regions immediately flanking promoters and enhancers feature compactly-ordered, regularly spaced nucleosomes (B. Lai, et al., Nature. 562, 281-285 (2018), K. Struhl, E. Segal, Nat. Struct. Mol. Biol. 20, 267-273 (2013)). However, while Fiber-seq data show shorter NR lengths within nucleosome arrays flanking both promoter and distal DHSs, these regions exhibited markedly greater architectural heterogeneity than nucleosome arrays further from DHSs (Fig. 2H). As such, nucleosome compaction does not appear to create or enforce architectural uniformity; rather, chromatin assembly surrounding regulatory regions appears to be a highly dynamic process at the level of individual templates.

FIGS. 2A-2H. Fiber-seq exposes base-pair resolution maps of individual chromatin fiber architecture. Fig. 2A. Fiber-seq schematic. Fig. 2B. Percentage of chromatin fibers with m6A-methylated bases from DNA isolated from untreated and Hia5-treated S2 nuclei subjected to PacBio CCS. Fig. 2C. Genomic loci comparing the relationship between DNaseI-seq, MTase-seq and Fiber-seq. Individual PacBio reads/chromatin fibers are marked with grey lines and m6A methylated bases are marked in purple dashes. Insert of DHS colored by base with m6A-sensitive bases in grey (e.g. all A/T residues) and m6A-methylated bases in purple. Fig. 2D. Beanplots displaying the relationship between DNaseI-seq signal and the Fiber-seq m6A signal at each DHS. Pearson correlation performed across all DHSs. *p-value <0.001 (Wilcoxon test). Fig. 2E. Histogram of MADs widths for all MADs identified outside of DHSs (grey), within TSS-distal DHSs (blue) and within promoter DHSs (green). Box-and-whisker plots for each shown below. *p-value <0.001 (Wilcoxon test). Fig. 2F. Heatmap of individual m6A marks surrounding MADs 5-100bp in length that do not overlap a DHS (top). Histogram of above, demonstrating strong protection from methylation at nucleosome-bound bases (bottom). Fig. 2G. Histogram of all NR lengths (left) as well as the number of NRs identified on individual chromatin fibers (right). Fig. 2H. Histogram of average NR lengths per fiber (left) and average bp difference in NR lengths per fiber (right), for fibers not containing a DHS (grey), containing a TSS-distal DHS (blue) or containing a promoter DHS (green). Box-and-whisker plots below. *p-value <0.001 (Wilcoxon test).

### EXAMPLE 3: CO-ACTUATION OF NEIGHBORING REGULATORY ELEMENTS ON THE SAME CHROMATIN FIBER

How regulatory information encoded in genomic DNA is activated by transcription factors and accessory proteins is a question of fundamental importance for understanding cell state and fate decisions (A. B. Stergachis, et al., Cell. 154, 888-903 (2013)), and defining the mechanism(s) by which genetic variation within regulatory DNA impacts phenotypic traits and disease risk (M. T. Maurano, R. et al., Science (80). 337, 1190-5 (2012)). An answer to long-standing question was sought: is regulatory DNA actuated in an all-or-none fashion on any given template (in place of a canonical nucleosome), or are actuated elements constitutively present as alternative structures with intermediate DNA accessibility mediated by intermittent variable nucleosome occupancy events? If the former, then the major mechanism by which genetic variation in a given regulatory region becomes penetrant is likely to involve altering the frequency with which its cognate region is actuated, rather than creating an alternative regulatory structure. Current approaches that aggregate data from populations of cells (R. E. Thurman, et al., 2012 *(supra))* are not able to address whether any regulatory element is actuated in an all-or-none fashion on individual chromatin templates, nor are single cell sampling approaches since these yield extremely sparse data and cannot continuously interrogate any region of chromatin on an allele-specific basis (J. D. Buenrostro, et al., Nature. 523, 486-490 (2015)).

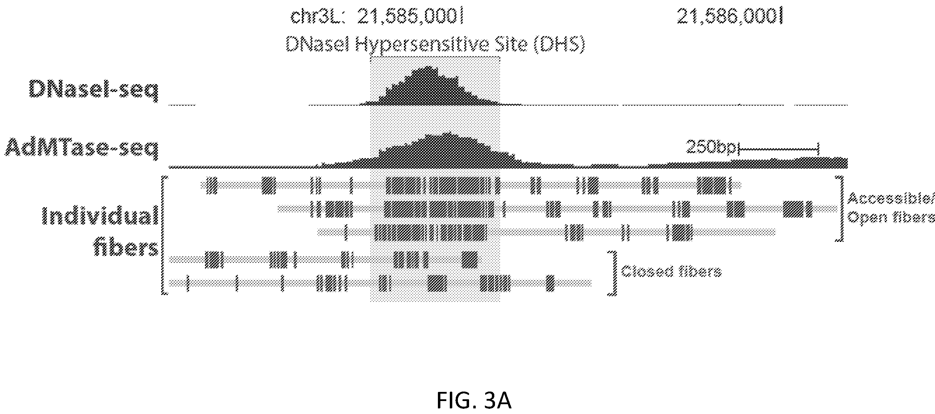

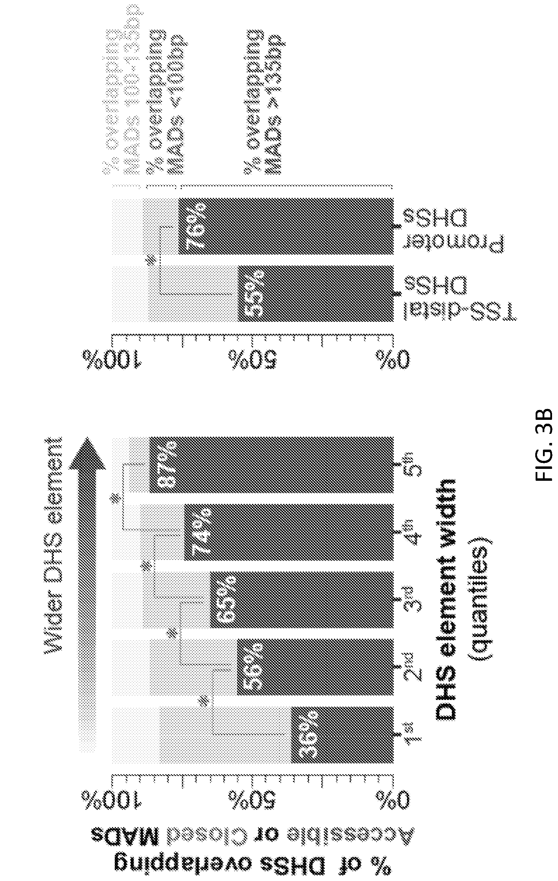

Across all 14,432 S2 cell DHSs with multiple overlapping Fiber-seq reads (mean of 5 high-quality reads per DHS), only 64% of overlapping chromatin fibers showed coinciding MADs indicative of an open state, with the remainder showing nucleosome demarcation indicative of a closed state (Fig. 3A). Wider DHSs showed higher probability of adopting an accessible/open state (Fig. 3B), consistent with the concept that cooperative binding of additional TFs along a DNA segment can more effectively compete with nucleosomes for DNA occupancy (C. C. Adams, J. L. Workman, Mol. Cell. Biol. 15, 1405-21 (1995), J. A. Miller, J. Widom, Mol. Cell. Biol. 23, 1623-32 (2003), L. A. Mirny, Proc. Natl. Acad. Sci. U. S. A. 107, 22534-22539 (2010)). Analysis of extended elements >500bp in length revealed that although 85% of the chromatin fibers overlapping these DHSs adopted an accessible state, 65% of these accessible fibers were punctuated by one or more co-occupying nucleosomes, with a high degree of positional variability recorded on different fibers. It was concluded from Fiber-seq data that regulatory DNA accessibility is predominantly actuated via an all-or-none process, and that at the level of individual chromatin fibers, most regulatory DNA exists in one of two states, accessible or inaccessible, with large elements additionally modulated by co-occupying nucleosomes.

The question of whether actuation of DNA accessibility at one regulatory element can influence the behavior of neighboring elements on the same chromatin fiber was addressed. Regulatory DNA is highly clustered along the genome, and many control regions appear to be organized as composites of multiple individual elements such as locus control regions/ "super-enhancers" functioning over entire gene loci (e.g., the beta-globin locus control region) or gene-specific control clusters (e.g., the BCL11A enhancer region) (W. A. Whyte, et al., Cell. 153, 307-319 (2013), P. Diaz, et al., Immunity. 1, 207-17 (1994), L. Madisen, M. Groudine, Genes Dev. 8, 2212-26 (1994), F. Grosveld, et al., Cell. 51, 975-85 (1987)). If the actuation of one regulatory element potentiates that of nearby elements on the same chromatin fiber, it would provide a mechanistic basis clusters of gene regulatory elements and indicate a level of cis-integrated function not accounted for in current models of the architecture and evolution of gene regulation. It would also broaden the potential impact of regulatory genetic variation via knock-on effects on neighboring elements.

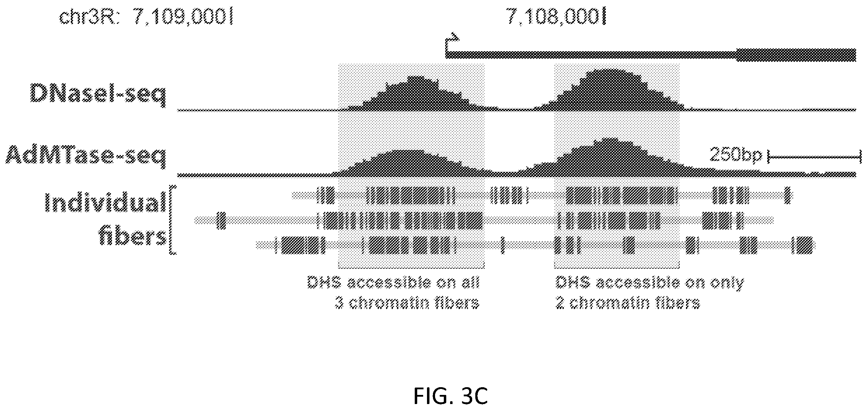

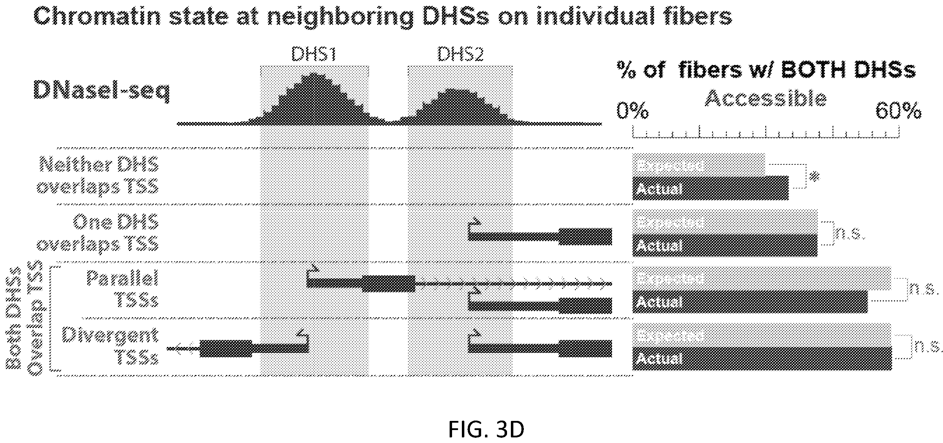

6% of Fiber-seq reads overlapped multiple DHSs, enabling quantification of co-actuation of regulatory DNA accessibility on the same chromatin fiber (Fig. 3C). Co-actuation was observed for pairs of distal elements; pairs of promoters; and combinations thereof, indicating that neighboring regulatory DNA elements can be actuated along the same chromatin fiber (Fig. 3D). Of these, neighboring distal regulatory elements were significantly enriched over background for being co-actuated on the same fiber (Fig. 3D), indicating that the actuation of regulatory DNA accessibility at one distal element potentiates accessibility at neighboring elements. These results thus provide a physicochemical basis for observed clustering of distal regulatory elements in animal genomes, and suggest that genetic variants impacting regulatory DNA accessibility and function are likely to create local knock-on effects in cis, broadening their potential impact in ways not captured by current genome interrogation technologies.

FIGS. 3A-3D. Co-actuation of neighboring regulatory elements on the same chromatin fiber. Fig. 3A. Genomic locus comparing the relationship between DNaseI-seq, MTase-seq and Fiber-seq at a DHS exposes overlapping chromatin fibers with open versus closed chromatin at that DHS. Fig. 3B. The proportion of DHSs that overlap accessible versus closed fibers for DHSs divided based on their width (left), or proximity to a TSS (right). *p-value <0.01 (z-test). Fig. 3C. Genomic locus comparing the relationship between DNaseI-seq, MTase-seq and Fiber-seq at neighboring DHSs. Fig. 3D. For chromatin fibers encompassing two DHSs, shown is the percentage of fibers containing accessible MADs at both DHSs for different classes of elements compared to the expected percentage. *p-value <0.01 (z-test).

### EXAMPLE 4: IMPACT OF REGULATORY DNA ACTUATION ON NUCLEOSOME POSITIONING

Nucleosome positioning is critical for gene regulation and is specified by a combination of factors including DNA sequence; competitive occupancy of sequence-specific DNA binding proteins creating a boundary; the action of nucleosome remodelers; and interactions with RNA polymerases (K. Struhl, E. Segal, Nat. Struct. Mol. Biol. 20, 267-273 (2013)). The relative contribution of these factors is currently unclear on a global level, and has not been possible to study at specific genomic locations. Prior analyses based on bulk cell data (S. Baldi, et al., Mol. Cell. 72, 661-672.e4 (2018), G.C. Yuan, et al., Science (80). 309, 626-630 (2005), C. Jiang, B. F. Pugh, Nat. Rev. Genet. 10, 161-172 (2009)) have indicated that nucleosomes surrounding accessible promoters are generally well-positioned, with nucleosome positioning surrounding distal regulatory elements being less well defined. However, whether this positioning is due to a boundary condition imposed by factor-occupied (and hence accessible) regulatory DNA is not clear.

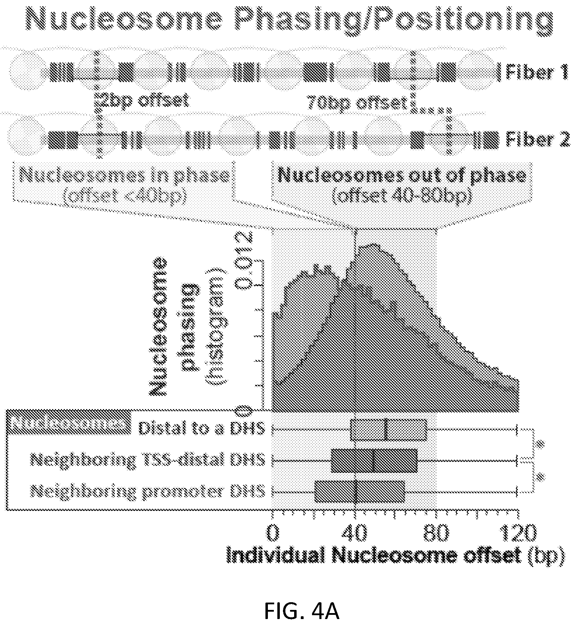

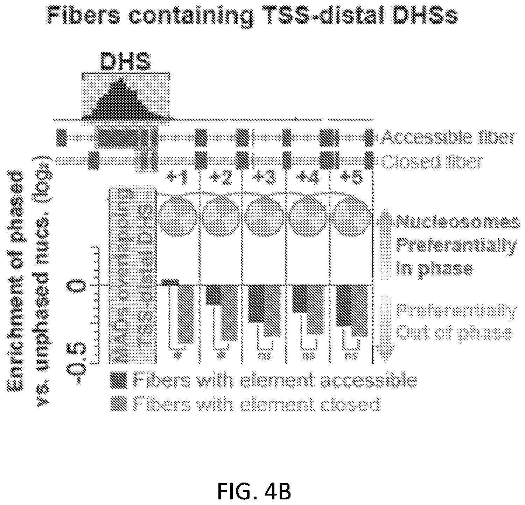

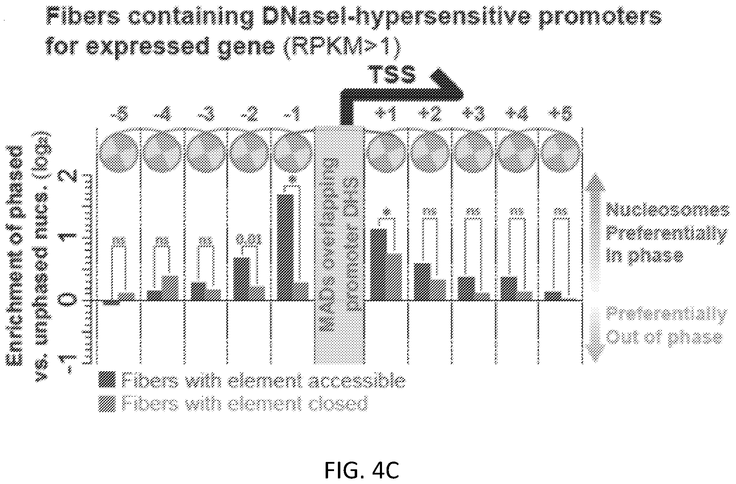

We reasoned the boundary model of nucleosome positioning could be tested directly by comparing nucleosome positions surrounding a regulatory element on overlapping fibers in which the regulatory element is in an accessible state vs. overlapping fibers in which the regulatory element is in the alternate nucleosome-occupied (i.e. closed) state. While nucleosomes surrounding DHSs were collectively well-positioned (Fig. 4A), analysis of single fiber data showed that these well-positioned nucleosomes largely originated from fibers in which the regulatory element is in an accessible state at either distal elements (Fig. 4B) or upstream of DNase I-hypersensitive promoters (Fig. 4C), indicating that nucleosome positioning at these locations is largely dependent on the actuation of regulatory DNA, not the DNA sequence itself. In contrast, nucleosomes downstream of DNaseI-hypersensitive promoters were well-positioned independent of whether the promoter was in an accessible or closed state (Fig. 4C). As such, in most instances nucleosome positioning appears to result from a boundary condition imposed by actuation of regulatory DNA on individual chromatin templates.

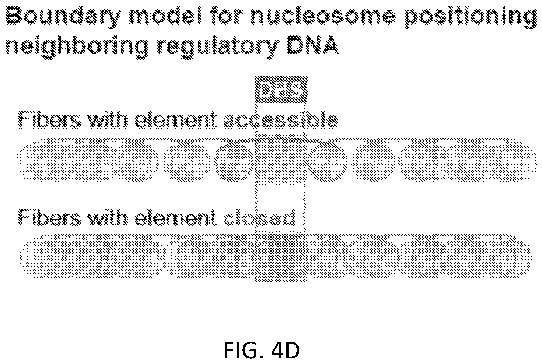

FIGS. 4A-4D. Impact of regulatory DNA actuation on nucleosome positioning. Fig. 4A. Schematic demonstrating the calculation of nucleosome phasing/positioning across overlapping reads as well as a histogram and box-and-whisker plots of individual nucleosome offsets for nucleosomes located on different classes of reads. *p-value <0.001 (Wilcoxon test). Figs. 4B-4C. Enrichment of nucleosomes in phase versus out of phase for nucleosomes neighboring TSS-distal DHSs (Fig. 4B) and promoter DHSs for expressed genes (Fig. 4C) divided based on whether the chromatin fiber contains an accessible MADs (red) versus closed MADs (grey) overlapping the DHS. *p-value <0.01; ns = p-value >0.05 (z-test). Fig. 4D. Schematic demonstrating the boundary model for nucleosome placement surrounding regulatory elements.

### EXAMPLE 5: CONSERVATION OF CHROMATIN ARCHITECTURE BETWEEN DROSOPHILA AND HUMANS

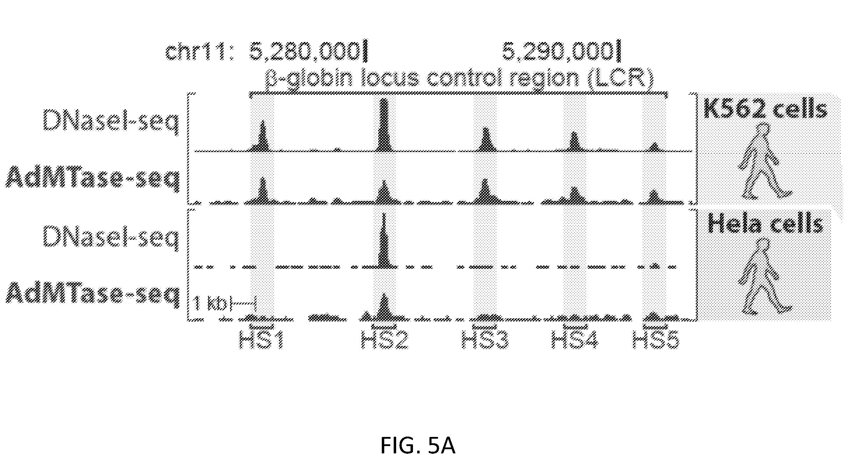

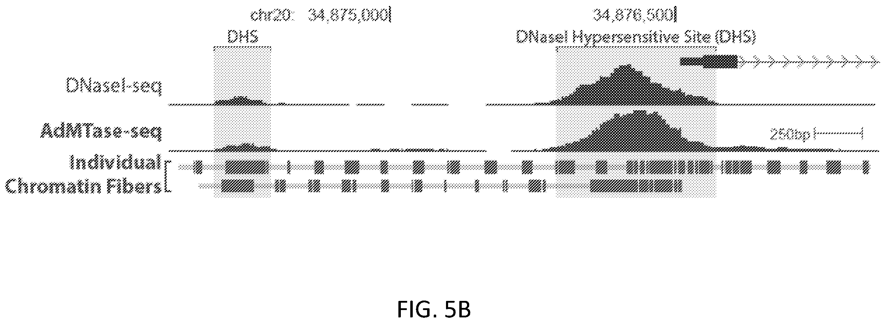

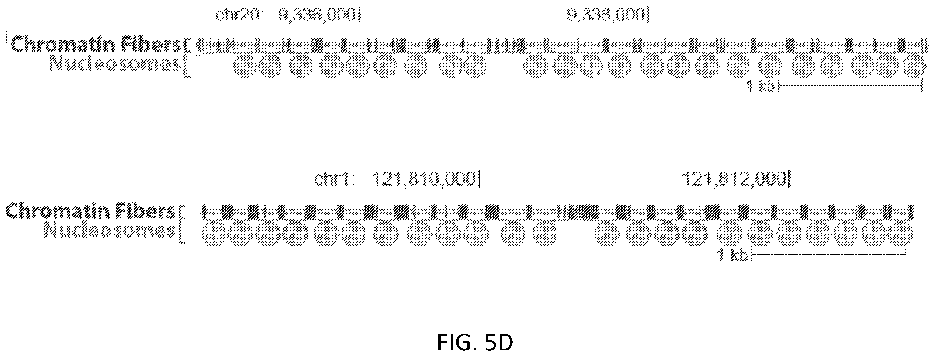

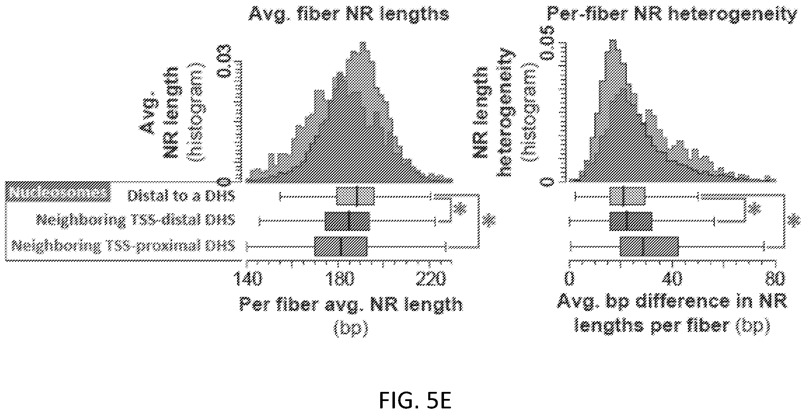

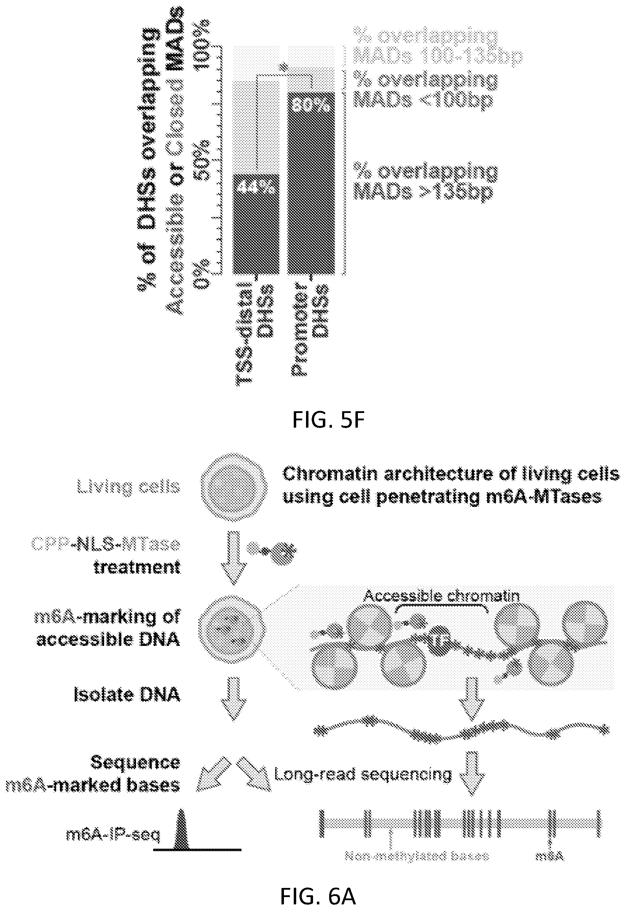

We next sought to identify if these chromatin features are conserved between drosophila and humans. After validating that m6A-MTases can selectivity mark cell-type specific accessible DNA across human cell types (Fig. 5A) we created chromatin stencils in human K562 cells by treating their nuclei with m6A-MTase followed by Pacific Biosystems CCS single molecule DNA sequencing. This resulted in the robust methylation of both accessible regulatory elements and internucleosomal linker regions (Figs. 5B-C) with the aggregate number of m6A-marked bases overlapping a DHSs mirroring the aggregate density of DNase I cleavages obtained from bulk nuclei (mean of 0.3 high-quality reads per DHS) and the average high-quality fiber yielding >8 well-demarcated nucleosomes (Fig. 5D). Consistent with our drosophila findings (Fig. 2H), we observed that nucleosome arrays flanking both promoter and distal DHSs in K562 cells had shorter NR lengths yet exhibited markedly greater architectural heterogeneity than nucleosome arrays further from DHSs (Fig. 5E). We similarly found that regulatory DNA accessibility in K562 cells is predominantly actuated via an all-or-none process (Fig. 5F) and that nucleosomes surrounding both promoter and distal DHSs are collectively well-positioned, indicating that these are likely universal features of regulatory DNA actuation and surrounding nucleosome positioning and compaction.

In conclusion, it is shown that it is possible to stencil chromatin and regulatory architecture onto DNA templates at nucleotide resolution, and to integrate this with long-read single-molecule DNA analysis to delineate the primary structure of individual chromatin fibers (Fiber-seq). Because the actuation of regulatory DNA is highly cell-selective, it should be possible to decompose single molecule data from complex cell mixtures into regulatory template states deriving from constituent cell sub-populations. As both read lengths and throughput further increase, it should be possible in the near future to transcribe the primary regulatory architecture of large gene loci, and to assemble entire chromatin haplotypes by combining Fiber-seq with accurate base calling of genetic variants. By simultaneously mapping both the genetic (primary sequence) and epigenetic (chromatin architecture) states of individual regulatory alleles, sequencing individual chromatin fibers provides a unifying tool for directly analyzing the functional impact of both rare and common regulatory DNA variation

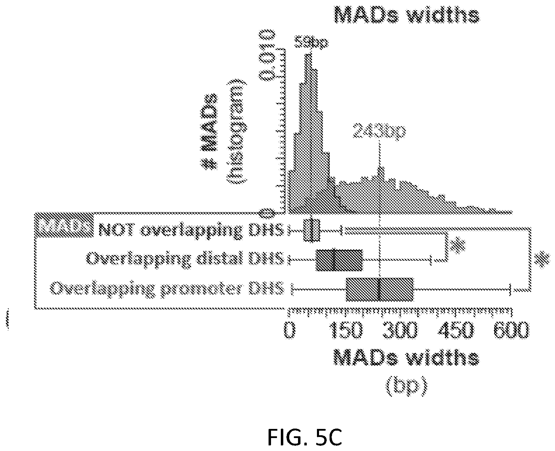

FIGS. 5A-5E. Conservation of chromatin architecture between drosophila and humans. Figs. 5A-5B. Comparison of DNaseI-seq signal and MTase-seq signal at the human β-globin locus control region in both Hela cells and K562 cells (Fig. 5A) as well as comparison with Fiber-seq data in K562 cells (Fig. 5B). Fig. 5C. Histogram of MADs widths for all MADs identified outside of DHSs (grey), within TSS-distal DHSs (blue) and within promoter DHSs (green). Box-and-whisker plots for each shown below. *p-value <0.001 (Wilcoxon test). Fig. 5D. Histogram of average NR lengths per fiber (left) and average bp difference in NR lengths per fiber (right), for fibers not containing a DHS (grey), containing a TSS-distal DHS (blue) or containing a promoter DHS (green). Box-and-whisker plots below. *p-value <0.001 (Wilcoxon test). Fig. 5E. The proportion of DHSs that overlap accessible versus closed fibers for DHSs divided based on their proximity to a TSS. *p-value <0.01 (z-test).

### EXAMPLE 6: IDENTIFICATION OF IN VIVO CHROMATIN ARCHITECTURES USING A CELL PENETRATING PEPTIDE (CPP)-TAGGED M6A-MTASE

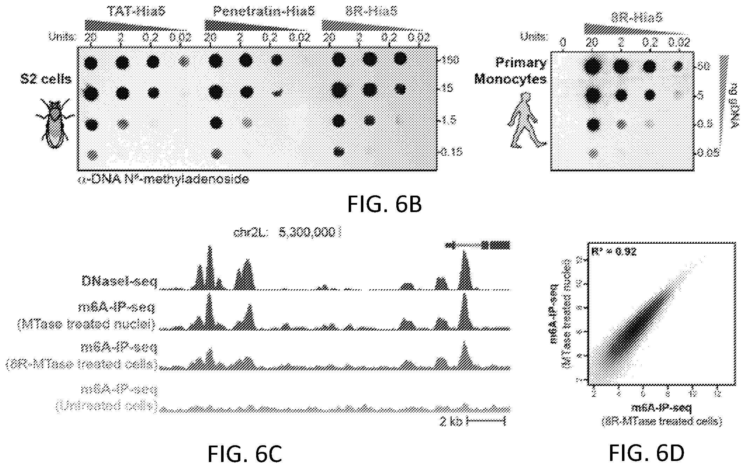

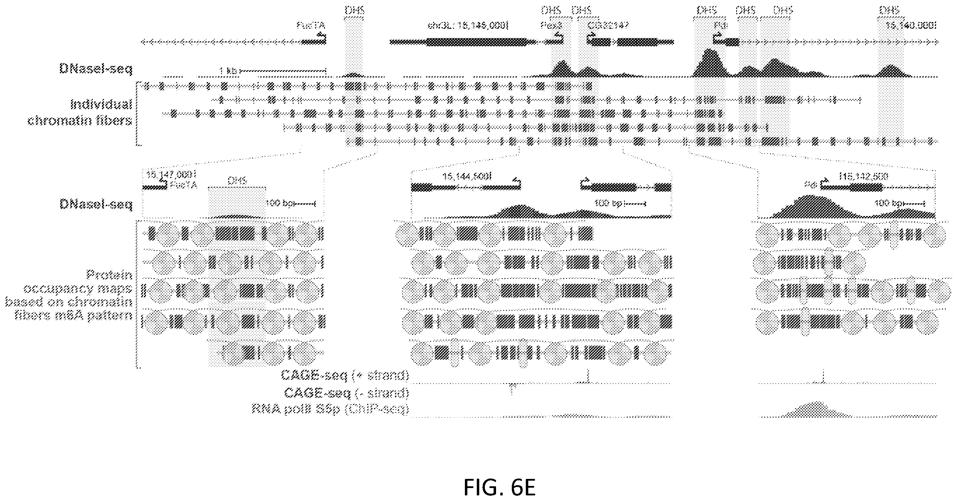

A modified m6A-MTase was generated that contains the m6A-MTase Hia5 conjugated to a cell penetrating peptide (CPP) and a nuclear localization sequence (NLS). Specifically, the CPP-tag enables the m6A-MTase to penetrate through the plasma membrane of living cells, and the NLS-tag enables the MTase to subsequently be shuttled into the nucleus of the cell. Single-molecule chromatin fiber sequencing with direct base modification determination of DNA isolated after treating living cells with this reagent (i.e. *in vivo* Fiber-seq) thus enables the identification of the chromatin architecture and dynamics of cells while they are still living (Fig. 6A).

Multiple CPP tags can be used for this approach. The CPP-tags TAT, 8-Arginine (8R), and Penetratin all demonstrated similar efficiency (Fig. 6B). This approach was successfully applied to primary blood cells (Fig. 6B). In vivo Fiber-seq profiles mirror those obtained from isolated nuclei, with the added benefit of not needing a nuclear isolation step, and also enabling quantification of chromatin dynamics that are occurring in vivo (Figs. 6C-6E).

### EXAMPLE 7: IDENTIFICATION OF FUNCTIONAL GENE REGULATORY DNA ALTERATIONS USING FIBER-SEQ

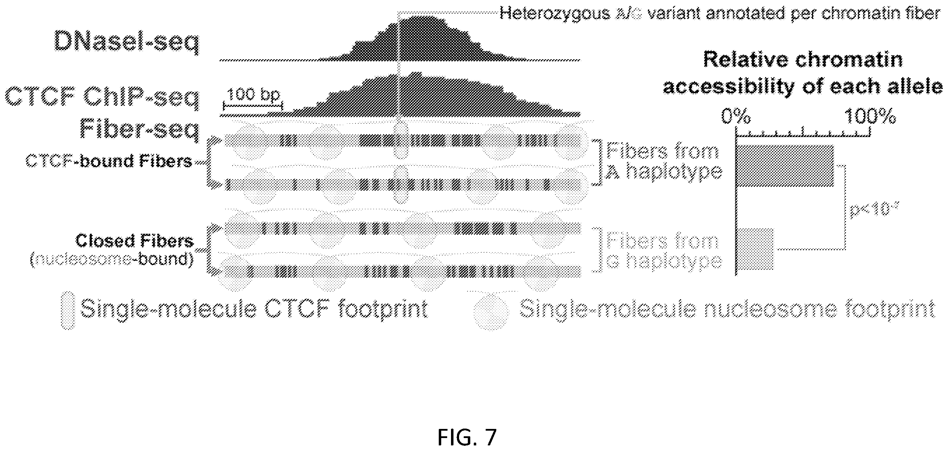

Functional regulatory DNA alterations can be readily elucidated using Fiber-seq by combining the per-molecule m6A-marked chromatin architectures with underlying per-molecule high quality DNA sequencing information obtained during single molecule sequencing. As shown in the Fig. 7, Fiber-seq was performed in primary human CD4+ cells, and functional regulatory DNA variants were identified based on the impact that these DNA variants had on the overlying chromatin architecture on those fibers.

### EXAMPLE 8: VISUALIZATION OF METHYLATED ADENINE (M6A) SITES IN SITU

An imaging assay to visualize methylated adenine (m6A) sites *in situ,* i.e. in intact mammalian cells was developed.

K562 cells were treated with rinsed 1x with PBS, and the cell pellet was resuspended in Buffer A. Resuspended cells were then permeabilized with 0.1% IGEPAL for 5 minutes on ice. The cell sample was pelleted and resuspended in buffer A, following which the cells were treated with 0U, 1U, or 40U of the Hia5 adenine methyl transferase enzyme, immediately seeded onto a PLL-coated glass surface at a density of 100,0000 cells per ml, incubated at 37°C for 15 minutes, and thereafter fixed with an excess solution of 4% paraformaldehyde for 10 minutes at room temperature. Fixed cells were washed 2x with PBS, permeabilized with 0.25% Triton for 10 minutes, then treated with RNaseA for 30 minutes at 37°C, blocked with 2% BSA for 1 hour, and then labeled with m6A antibodies (ABE572, ABE572-I, SAB5600251) using standard immunofluorescence procedures. Post m6A labeling, cells were counterstained with DAPI, and mounted with Prolong Gold antifade. Cells were then imaged in 3D by epifluorescence microscopy using a 60x 1.4 NA oil immersion objective. Cell images were deconvolved to remove out of focus blur, and then processed to delineate individual cell nuclei and m6A labeled nuclear regions.

Labeling with anti-m6A antibody revealed defined, punctate staining which increases with dose of Hia5 adenine methyl transferase enzyme.

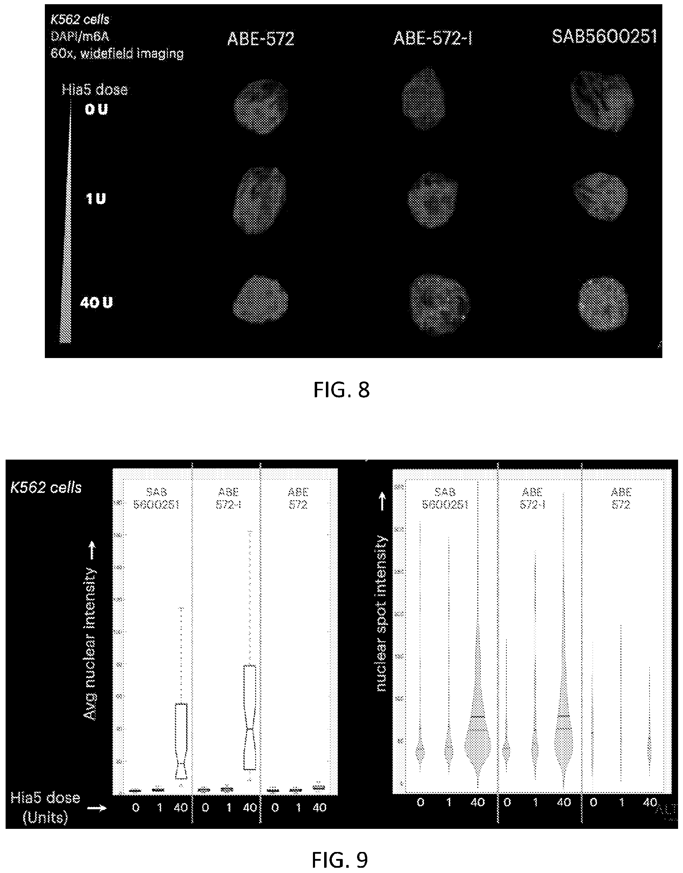

Fig. 8. Representative images of K562 cell nuclei stained with DAPI and m6A show a punctate m6A pattern that increases with Hia5 dose for all three tested m6A antibodies.

Dose-dependent increase in nuclear m6A signal was reflected in both overall nuclear expression as well as in individual puncta.

Fig. 9. Box and violin plots illustrate the Hia5 dose-dependent increase in the overall nuclear m6A signal (left) as well as in the intensity of individual puncta (right).

Visualization of genomic regions labeled with m6A would provide insight into the spatial organization of the accessible genome at the single cell level, and thereby enable research inquiries into the structure-function interrelationships of regulatory DNA. The visualization can also be used to profile diseased vs. normal cells.

It is to be understood that the terminology used herein is for the purpose of describing particular embodiments only, and is not intended to be limiting, since the scope of the present invention will be limited only by the appended claims.

Furthermore, all examples and conditional language recited herein are principally intended to aid the reader in understanding the principles of the invention and the concepts contributed by the inventors to furthering the art, and are to be construed as being without limitation to such specifically recited examples and conditions. The scope of the present invention, therefore, is not intended to be limited to the exemplary embodiments shown and described herein. Rather, the scope present invention is embodied by the appended claims

## Claims

1. A method for identifying at single nucleotide resolution regions of genomic DNA bound to a protein, the method comprising:
a contacting step consisting of contacting genomic DNA with N⁶-adenine methyltransferase (m6 A-MTase), wherein the m6 A-MTase causes methylation of adenine residues in regions of the genomic DNA not bound to a protein, wherein the m6 A-MTase is Hia5;
conducting single-molecule long-read sequencing of the contacted genomic DNA to detect locations in the genomic DNA lacking methylated adenine residues to identify at single nucleotide resolution regions of genomic DNA bound to a protein.

2. The method of claim 1, wherein the contacting comprises contacting isolated genomic DNA with the m6 A-MTase.

3. The method of claim 1, wherein the contacting comprises contacting a cell comprising the genomic DNA.

4. The method of claim 3, wherein the contacting comprises contacting the cell with the m6 A-MTase fused to a cell penetrating peptide that renders the m6 A-MTase plasma membrane permeable.

5. The method of any one of claims 1-4, wherein the sequencing is conducted on a stretch of genomic DNA that is at least 1 kilobase (kb) long or at least 3 kb long.

6. The method of any one of claims 1-5, wherein the sequencing comprises translocating the genomic DNA through a nanopore, and optionally ligating one or more nanopore sequencing adapters to one or more ends of the genomic DNA, conducting multiple rounds of re-sequencing, and/or detecting a signal indicative of a methylated adenine.

7. The method of any one of claims 1-6, wherein the sequencing comprises circular consensus sequencing, optionally wherein the sequencing comprises single molecule real-time (SMRT) circular consensus sequencing (CCS).

8. The method of any one of claims 1-7, wherein the genomic DNA is from a mammalian cell, optionally wherein the genomic DNA is from a cancer cell.

9. The method of any one of claims 1-8, wherein the genomic DNA is from a normal cell or from a test cell and the method further comprises generating a chromatin accessibility map for the region of genomic DNA sequenced, wherein the map indicates regions of chromatin not bound to the protein and hence accessible to the m6 A-MTase and regions of the chromatin bound to the protein and hence inaccessible to the m6 A-MTase, optionally comparing the chromatin accessibility map for the test cell to that of the normal cell, wherein the test cell and the normal cell are of the same cell type and comparing the genomic DNA sequences of the test and normal cells, wherein presence of a difference in chromatin accessibility maps indicates a change in chromatin architecture in the test cell, wherein presence of a difference in genomic DNA sequence in absence of a difference in chromatin accessibility maps indicates that the sequence difference is not associated with a change in chromatin structure, and wherein presence of a difference in genomic DNA sequence and of a difference in chromatin accessibility maps indicates the sequence difference is associated with a change in chromatin structure, optionally wherein the normal cell and test cell are epithelial cells, white blood cells, glial cells, osteoblasts, or chondrocytes.

10. The method of claim 9, wherein the test cell is a cell from a subject, optionally wherein the subject has or is suspected of having a disease or wherein the disease is cancer.

11. The method of claim 9 or 10, further comprising generating a database comprising information regarding chromatin accessibility map, the underlying genomic DNA sequence, and correlation, if any, to a condition or disease, optionally wherein the chromatin accessibility map encompasses at least 10%, at least 30%, at least 50%, or at least 80% of a chromatin, or wherein the chromatin accessibility map encompasses at least 10%, at least 20%, at least 30%, at least 50%, or at least 80% of the genome of the cell.

12. The method of any one of claims 1-11, wherein the protein comprises nucleosomes, a transcriptional repressor, or a transcriptional activator.

13. A kit for use in the method of any one of claims 1-12, the kit comprising:
an enzyme consisting of a m6 A-MTase, wherein the m6 A-MTase is Hia5;
sequencing adapters; and
instructions for the use.

## Patentansprüche

1. Verfahren zur Identifizierung von an ein Protein gebundenen Bereichen genomischer DNA mit Einzelnukleotidauflösung, wobei das Verfahren Folgendes umfasst:
einen Kontaktierungsschritt, bestehend aus dem Kontaktieren genomischer DNA mit N⁶-Adenin-Methyltransferase (m6 A-MTase), wobei die m6 A-MTase eine Methylierung von Adeninresten in Bereichen der genomischen DNA bewirkt, die nicht an ein Protein gebunden sind, wobei es sich bei der m6 A-MTase um Hia5 handelt;
Durchführen einer Einzelmolekül-Sequenzierung mit langer Ableselänge der kontaktierten genomischen DNA, um Stellen in der genomischen DNA zu erkennen, denen methylierte Adeninreste fehlen, um Bereiche der genomischen DNA, die an ein Protein gebunden sind, mit Einzelnukleotidauflösung zu identifizieren.

2. Verfahren nach Anspruch 1, wobei das Kontaktieren Inkontaktbringen von isolierter genomischer DNA mit der m6 A-MTase umfasst.

3. Verfahren nach Anspruch 1, wobei das Kontaktieren Inkontaktbringen einer Zelle umfasst, die die genomische DNA umfasst.

4. Verfahren nach Anspruch 3, wobei das Kontaktieren Inkontaktbringen der Zelle mit der m6 A-MTase umfasst, die mit einem zellpenetrierenden Peptid fusioniert ist, das die Plasmamembran der m6 A-MTase permeabel macht.

5. Verfahren nach einem der Ansprüche 1-4, wobei die Sequenzierung an einem Abschnitt genomischer DNA durchgeführt wird, der mindestens 1 Kilobase (kb) lang oder mindestens 3 kb lang ist.

6. Verfahren nach einem der Ansprüche 1-5, wobei die Sequenzierung Translozieren der genomischen DNA durch eine Nanopore und optional Ligieren eines oder mehrerer Nanoporen-Sequenzierungsadapter an ein oder mehrere Enden der genomischen DNA sowie das Durchführen mehrerer Resequenzierungsrunden und/oder Nachweisen eines Signals, das auf ein methyliertes Adenin hinweist, umfasst.

7. Verfahren nach einem der Ansprüche 1-6, wobei die Sequenzierung eine zirkuläre Konsensussequenzierung umfasst, wobei die Sequenzierung optional eine zirkuläre Einzelmolekül-Echtzeit- (SMRT) Konsensussequenzierung (CCS) umfasst.

8. Verfahren nach einem der Ansprüche 1-7, wobei die genomische DNA aus einer Säugetierzelle stammt, wobei die genomische DNA optional aus einer Krebszelle stammt.

9. Verfahren nach einem der Ansprüche 1-8, wobei die genomische DNA aus einer normalen Zelle oder einer Testzelle stammt und das Verfahren weiter Erstellen einer Chromatinzugänglichkeitskarte für den sequenzierten Bereich der genomischen DNA umfasst, wobei die Karte Bereiche des Chromatins, die nicht an das Protein gebunden und daher für die m6 A-MTase zugänglich sind, und Bereiche des Chromatins anzeigt, die an das Protein gebunden und daher für die m6 A-MTase unzugänglich sind, wobei die Chromatinzugänglichkeitskarte der Testzelle optional mit der der normalen Zelle verglichen wird, wobei die Testzelle und die normale Zelle vom gleichen Zelltyp sind und die genomische DNA-Sequenz der Test- und der normalen Zelle verglichen werden, wobei das Vorhandensein eines Unterschieds in den Chromatinzugänglichkeitskarten eine Veränderung der Chromatinarchitektur in der Testzelle anzeigt, wobei das Vorhandensein eines Unterschieds in der genomischen DNA-Sequenz ohne einen Unterschied in den Chromatinzugänglichkeitskarten anzeigt, dass der Sequenzunterschied nicht mit einer Veränderung der Chromatinstruktur verknüpft ist, und wobei das Vorhandensein eines Unterschieds in der genomischen DNA-Sequenz und eines Unterschieds in den Chromatinzugänglichkeitskarten anzeigt, dass der Sequenzunterschied mit einer Veränderung der Chromatinstruktur verknüpft ist, wobei es sich bei der normalen Zelle und der Testzelle optional um Epithelzellen, weiße Blutkörperchen, Gliazellen, Osteoblasten oder Chondrozyten handelt.

10. Verfahren nach Anspruch 9, wobei es sich bei der Testzelle um eine Zelle eines Probanden handelt, wobei der Proband optional an einer Krankheit leidet oder der Verdacht besteht, dass er an einer solchen leidet, oder wobei es sich bei der Krankheit um Krebs handelt.

11. Verfahren nach Anspruch 9 oder 10, weiter umfassend Erstellen einer Datenbank, die Informationen über die Chromatinzugänglichkeitskarte, die eigentliche genomische DNA-Sequenz und, falls vorhanden, eine Korrelation zu einem Zustand oder einer Krankheit umfasst, wobei die Chromatinzugänglichkeitskarte optional mindestens 10%, mindestens 30%, mindestens 50%, oder mindestens 80 % eines Chromatins beinhaltet, oder wobei die Chromatinzugänglichkeitskarte mindestens 10%, mindestens 20%, mindestens 30%, mindestens 50%, oder zumindest 80 % des Genoms der Zelle beinhaltet.

12. Verfahren nach einem der Ansprüche 1-11, wobei das Protein Nukleosomen, einen Transkriptionsrepressor oder einen Transkriptionsaktivator umfasst.

13. Kit zur Verwendung in dem Verfahren nach einem der Ansprüche 1-12, wobei das Kit Folgendes umfasst:
ein Enzym, bestehend aus einer m6 A-MTase, wobei es sich bei der m6 A-MTase um Hia5 handelt;
Sequenzierungsadapter; und
eine Gebrauchsanweisung.

## Revendications

1. Procédé d'identification de régions de résolution mononucléotidique d'ADN génomique liées à une protéine, le procédé comprenant :
une étape de mise en contact consistant à mettre en contact l'ADN génomique avec la N⁶-adénine méthyltransférase (m6 A-MTase), dans lequel la m6 A-MTase provoque la méthylation des résidus d'adénine dans les régions de l'ADN génomique non liées à une protéine, dans lequel la m6 A-MTase est la Hia5 ;
la réalisation d'un séquençage à longue lecture de molécule unique de l'ADN génomique mis en cotact pour détecter des emplacements dans l'ADN génomique dépourvus de résidus d'adénine méthylée à identifier au niveau de régions de résolution mononucléotidique d'ADN génomique liées à une protéine.

2. Procédé selon la revendication 1, dans lequel la mise en contact comprend la mise en contact de l'ADN génomique isolé avec la m6 A-MTase.

3. Procédé selon la revendication 1, dans lequel la mise en contact comprend la mise en contact avec une cellule contenant l'ADN génomique.

4. Procédé selon la revendication 3, dans lequel la mise en contact comprend la mise en contact de la cellule avec la m6 A-MTase fusionnée à un peptide de pénétration cellulaire qui rend perméable la membrane plasmique de la m6 A-MTase.

5. Procédé selon l'une quelconque des revendications 1-4, dans lequel le séquençage est effectué sur une portion d'ADN génomique d'au moins 1 kilobase (kb) de long ou d'au moins 3 kb de long.

6. Procédé selon l'une quelconque des revendications 1-5, dans lequel le séquençage comprend la translocation de l'ADN génomique à travers un nanopore, et éventuellement la ligature d'un ou plusieurs adaptateurs de séquençage nanopore à une ou plusieurs extrémités de l'ADN génomique, la réalisation de plusieurs cycles de reséquençage, et/ou la détection d'un signal indiquant la présence d'une adénine méthylée.

7. Procédé selon l'une quelconque des revendications 1-6, dans lequel le séquençage comprend un séquençage consensus circulaire, éventuellement dans lequel le séquençage comprend un séquençage consensus circulaire (CCS) en temps réel de molécules uniques (SMRT).

8. Procédé selon l'une quelconque des revendications 1-7, dans lequel l'ADN génomique provient d'une cellule de mammifère, éventuellement dans lequel l'ADN génomique provient d'une cellule cancéreuse.

9. Procédé selon l'une quelconque des revendications 1-8, dans lequel l'ADN génomique provient d'une cellule normale ou d'une cellule d'essai, et le procédé comprend en outre la génération d'une carte d'accessibilité de la chromatine pour la région d'ADN génomique séquencée, dans lequel la carte indique les régions de chromatine non liées à la protéine et donc accessibles à la m6 A-MTase, ainsi que les régions de chromatine liées à la protéine et donc inaccessibles à la m6 A-MTase, éventuellement la comparaison de la carte d'accessibilité de la chromatine de la cellule d'essai à celle de la cellule normale, dans lequel la cellule d'essai et la cellule normale sont du même type cellulaire, et la comparaison des séquences d'ADN génomique des cellules d'essai et normale, dans lequel la présence d'une différence dans les cartes d'accessibilité de la chromatine indique une modification de l'architecture de la chromatine dans la cellule d'essai, dans lequel la présence d'une différence dans la séquence d'ADN génomique en l'absence de différence dans les cartes d'accessibilité de la chromatine indique que la différence de séquence n'est pas associée à une modification de la structure de la chromatine, et dans lequel la présence d'une différence dans la séquence d'ADN génomique et d'une différence dans les cartes d'accessibilité de la chromatine indique que la différence de séquence est associée à une modification de la structure de la chromatine, éventuellement dans lequel la cellule normale et la cellule d'essai sont des cellules épithéliales, des globules blancs, des cellules gliales, des ostéoblastes ou des chondrocytes.

10. Procédé selon la revendication 9, dans lequel la cellule d'essai est une cellule provenant d'un sujet, éventuellement dans lequel le sujet présente ou est susceptible de présenter une maladie, ou dans lequel la maladie est un cancer.

11. Procédé selon la revendication 9 ou 10, comprenant en outre la génération d'une base de données comprenant des informations relatives à la carte d'accessibilité de la chromatine, à la séquence d'ADN génomique sous-jacente et à la corrélation éventuelle avec une affection ou une maladie, éventuellement dans lequel la carte d'accessibilité de la chromatine englobe au moins 10 %, au moins 30 %, au moins 50 %, ou au moins 80 % d'une chromatine, ou dans lequel la carte d'accessibilité de la chromatine englobe au moins 10 %, au moins 20 %, au moins 30 %, au moins 50 %, ou au moins 80 % du génome de la cellule.

12. Procédé selon l'une quelconque des revendications 1-11, dans lequel la protéine comprend des nucléosomes, un répresseur transcriptionnel ou un activateur transcriptionnel.

13. Kit pour utilisation selon le procédé de l'une quelconque des revendications 1-12, le kit comprenant :
une enzyme consistant en une m6 A-MTase, dans lequel la m6 A-MTase est la Hia5 ;
des adaptateurs de séquençage ; et
une notice d'utilisation.
